(19) Europäisches Patentamt
European Patent Office
Office européen des brevets

(11) Numéro de publication: **0 165 123**
**B1**

(12) # FASCICULE DE BREVET EUROPEEN

(45) Date de publication du fascicule du brevet: **08.08.90**

(51) Int. Cl.$^5$: **C 07 K 9/00,** A 61 K 37/02, A 61 K 9/50

(21) Numéro de dépôt: **85400921.4**

(22) Date de dépôt: **10.05.85**

(54) **Dérivés lipophiles de muramylpeptides ayant des propriétés d'activation des macrophages, compositions les contenant et procédé pour les obtenir.**

(30) Priorité: **11.05.84 FR 8407340**

(43) Date de publication de la demande:
**18.12.85 Bulletin 85/51**

(45) Mention de la délivrance du brevet:
**08.08.90 Bulletin 90/32**

(84) Etats contractants désignés:
**AT BE CH DE FR GB IT LI LU NL SE**

(56) Documents cités:
**EP-A-0 056 992**
**EP-A-0 102 319**
**EP-A-0 135 788**

(73) Titulaire: **ANVAR Agence Nationale de Valorisation de la Recherche**
**43, rue Caumartin**
**F-75436 Paris Cédex 09 (FR)**

(72) Inventeur: **Phillips, Nigel**
**114 Maywood Road**
**Pointe Claire Que H9R 3L8 (CA)**
Inventeur: **Audibert, Françoise**
**44 Rue Ybry**
**F-92200 Neuilly S/Seine (FR)**
Inventeur: **Bernard, Jean-Marie**
**7, Square Couperin Résidence du Parc St. Cyr**
**F-78330 Fontenay le Fleury (FR)**
Inventeur: **Chedid, Louis**
**18 Rue Gaston Caillavet**
**F-75015 Paris (FR)**
Inventeur: **Lefrancier, Pierre**
**46 Allée de la Mare l'Oiseau Chevry 2**
**F-91190 Gif S/Yvette (FR)**
Inventeur: **Level, Michel**
**24 rue Sibuet**
**F-75012 Paris (FR)**
Inventeur: **Parant, Monique**
**33 Rue Coulebarbe**
**F-75013 Paris (FR)**

**EP 0 165 123 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

(74) Mandataire: **Gutmann, Ernest et al**
**S.C. Ernest Gutmann - Yves Plasseraud 67,**
**boulevard Haussmann**
**F-75008 Paris (FR)**

**Description**

L'invention concerne des dérivés lipophiles de muramylpeptides ayant des propriétés d'activation des macrophages, et plus particulièrement des propriétés tumoricides. Elle concerne plus particulièrement des compositions contenant ces dérivés lipophiles de muramylpeptides, plus particulièrement de compositions à base de liposomes ou susceptibles de former des liposomes auxquels sont incorporés lesdits dérivés lipophiles de muramylpeptides.

Des dérivés lipophiles de muramylpeptides ont déjà été decrits dans les demandes de brevet européen EP—A—0.056.992, EP—A—0.102.319 et EP—A—0.135.788.

L'activation des macrophages est l'un des principaux mécanismes de l'activité antitumorale des immunomodulateurs: les macrophages activés sont capables de détruire des cellules tumorales syngénéiques non seulement *in vitro,* mais aussi *in vivo.*

Il a aussi été montré que les muramylpeptides pouvaient augmenter l'activité antitumorale des macrophages *in vitro.* Il n'en est pas de même *in vivo* lorsque ces substances sont utilisées en solution sailine. Cette inactivité est sans doute due au fait que les muramylpeptides pénètrent lentement dans les macrophages, sans doute par pinocytose fluide et que les muramylpeptides sont éliminés rapidement de l'organisme par le rein. Il résulterait alors de la conjonction de ces deux phénomènes que les muramylpeptides injectés en solution saline n'atteignent pas une concentration suffisante dans les macrophages pour pouvoir les activer.

Plusieurs solutions ont été envisagées pour remédier à ces inconvénients. En particulier, il a été montré que l'encapsulation des muramylpeptides dans des liposomes conférait aux macrophages une activité cytotoxique déjà importante in vitro et *in vivo.* FIDLER et ses collaborateurs ont développé cette approche par l'utilisation de liposomes multilamellaires composés de phosphatidylcholine (PC) et de phosphatidylsérine (PS) dans un rapport 7/3 et incluant du MDP; ils arrivent ainsi à cibler cet immunomodulateur vers les monocytes circulants qui se différencient, sous l'influence du MDP qu'ils ont endocyté, en macrophages activés (Canc. Res., *42,* 161—167 (1982)).

La composition des liposomes et leur nature (multilamellaires) permettent de les diriger, en particulier vers les capillaires de la circulation pulmonaire. Les monocytes qui les ont phagocytés migrent ensuite dans le poumon où il se différencient en macrophages actives: ces macrophages activés sont capables de détruire des métastases de tumeurs à tropisme pulmonaire comme le mélanome $B_{16}$ cherz la souris.

Mais l'utilisation de muramylpeptides solubles se heurte à de nombreux inconvénients: certains liposomes (en particulier ceux dont la composition favorise le ciblage vers les monocytes de la circulation pulmonaire) fuient", c'est-à-dire qu'ils perdent le soluté encapsulé: cette fuite est particulièrement marquée lorsque les liposomes PC/PS sont mis en présence de sérum. Ces fuites interdisent naturellement une bonne conservation de liposomes.

Cet inconvénient est partiellement surmonté par l'utilisation de liposomes multilamellaires. On peut en effet penser que les espaces interlamellaires les plus internes peuvent contenir ou limiter les fuites avant la phagocytose du liposome. Cette solution entraine cependant une perte de spécificité du ciblage. En effet l'utilisation de liposomes unilamellaires ou constitués de peu de lamelles pourrait présenter des avantages pour le ciblage vers d'autres organes que le poumon (G. POSTE et Coll., Cancer Res., *42,* 1412—1422 (1982)).

Pour y remédier, FIDLER et Coll. ont également déjà préconisé l'utilisation de dérivés lipophiles de la N-acétyl-muramyl-L-alanyl-D-isogutamine (MDP) ou de la N-acétyl-muramyl-L-alanyl-D-isoglutamyl-L-alanine (MTP), tels que le MTP-phosphatidyléthanolamine.

Il a également déjà été proposé de conjuguer le MDP ou le MTP à la phosphatidyléthanolamine ou à une molécule d'acide stéarique, en vue de stimuler l'activité cytotoxique des macrophages. Cependant, la préparation de liposomes contenant de tels dérivés ne va pas sans difficulté. En particulier, la formation de pellicules fines de lipides contenant ces dérivés de muramylpeptides et la transformation de ces fines pellicules de lipides en liposomes par des méthodes classiques ne conduisent pas toujours à des résultats reproductibles et les liposomes ne peuvent guère être conservés pendant des periodes prolongées.

En outre, il s'est avéré que certains dérivés de muramylpeptides contenant déjà certains groupes lipophiles pouvaient présenter des propriétés toxiques et/ou déstabilisatrices à légard des macrophages.

L'invention a pour but de remédier encore plus efficacement aux difficultés qui ont été évoquées ci-dessus, en particulier de fournir de nouveaux dérivés de MDP, possédant ne capacité importante d'activation des macrophages, et plus particulièrement de leur activité tumoricide *in vivo*, et ce plus particulièrement lorsqu'ils sont administrés sous forme de liposomes.

L'invention a encore pour but de fournir des dérivés lipophiles de muramylpeptides qui puissent être utilisés entre autres dans la préparation de liposomes les incorporant, ces liposomes se caractérisant alors simultanément par des propriétés de stimulation particulièrement élevées des macrophages et par une grande stabilité, tant en ce qui concerne les possibilités de stocker ces liposomes (ou des compositions pouvant être aisément transformées en liposomes) que leur absence d'action déstabilisatrice ou toxique à l'égard des macrophages.

Les dérivés de muramylpeptides selon l'invention sont essentiellement caractérisés par la formule générale suivante:

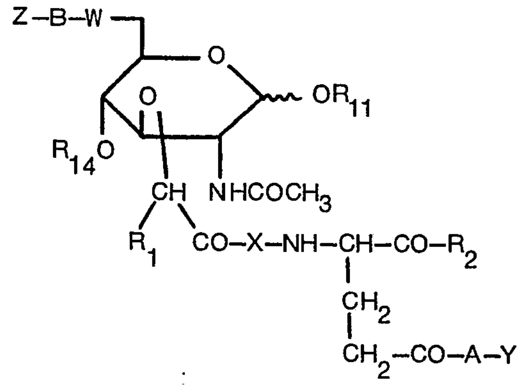

dans laquelle les substituants $R_1$, $R_2$, $R_{11}$, $R_{14}$, W, X, Y, A et B ont les significations suivantes:

$R_1$ est H ou $CH_3$,

$R_2$ est un groupe $—NH_2$, —OH ou un groupe —OV, avec V étant un groupe hydrocarboné comportant de 1 à 10 atomes de carbone,

$R_{11}$ est un atome d'hydrogène ou un groupe phénylamino,

$R_{14}$ est un atome d'hydrogène ou un groupe acyle comprenant de 1 à 5 atomes de carbone,

X est un résidu aminoacyle du groupe comprenant: alanyle, valyle, isoleucyle, norleucyle, leucyle, séryle, thréonyle, prolyle, glutaminyle, asparaginyle, méthionyle, tryptophanyle, phénylalanyle, tyrosyle, glycyle, étant entendu que ce résidu aminoacyle est éventuellement N-substitué par un groupe alcoyle inférieur, notamment méthyle;

Y a la même signification que $R_2$ ou est un groupe lipophile $—OCH_2—CHO(R_3)CH_2O(R_4)$ avec $R_3$ et $R_4$, respectivement identiques ou différents, étant des groupements acyle ou alkyle comprenant de 8 à 100 atomes de carbone;

Z a la même signification que $R_2$ ou est un groupe lipophile $—CO—CHO(R_3)CH_2O(R_4)$ avec $R_3$ et $R_4$, respectivement identiques ou différents, étant des groupements alkyle ou acyle comprenant de 8 à 100 atomes de carbone, les groupes méthylène contenus dans les groupes glycéryle, $R_3$ ou $R_4$, étant le cas échéant substitués par un groupe alcoyle inférieur, notamment méthyle; ètant cependant entendu que l'un au moins des deux groupes Y et Z est toujours constitué par le groupe lipophile correspondant;

W est un atome d'oxygène ou, lorsque Z est le susdit groupe lipophile, un groupe NH;

A et B sont soit des liaisons directes, soit des bras de pontage, notamment des groupes, respectivement identiques ou différents, comprenant respectivement de un à trois résidus aminoacyle, eux-mêmes identiques ou différents entre eux, ou encore un groupe

$—NH—(CH_2)_p—CO—$ avec des valeurs de p comprises entre 2 et 10, ou $—NH—CH_2—CH_2—O—$, et enfin l'un des groupes méthylène du résidu glutamyle étant, le cas échéant, substitué par un alcoyle inférieur, notamment méthyle.

Des composés préférés de l'invention sont caractérisés par la formule suivante:

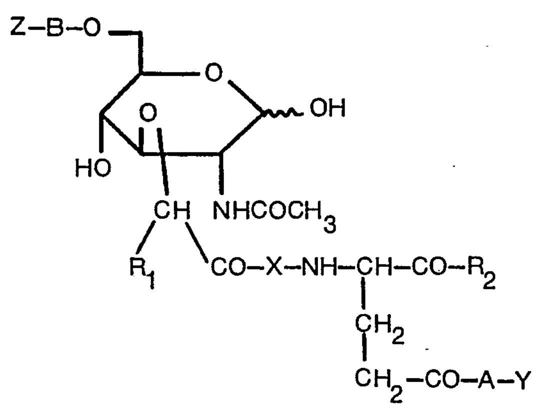

dans laquelle les substituants $R_1$, $R_2$, X, Y, A et B ont les significations suivantes:

$R_1$ est H ou $CH_3$,

$R_2$ est un groupe $—NH_2$, —OH ou un groupe —OV, avec V étant un groupe hydrocarboné comportant de 1 à 10 atomes de carbone,

X est un résidu aminoacyle du groupe comprenant: alanyle, valyle isoleucyle, norleucyle, leucyle,

séryle, thréonyle, prolyle, glutaminyle, asparaginyle, méthionyle, tryptophanyle, phénylalanyle, tyrosyle, glycyle,

Y a la même signification que $R_2$ ou est un groupe lipophile $OCH_2$—$CHO(R_3)CH_2O(R_4)$ avec $R_3$ et $R_4$, respectivement identiques ou différents, étant des groupements acyle comprenant de 8 à 100 atomes de carbone;

Z a la même signification que $R_2$ ou est un groupe lipophile —CO—$CHO(R_3)CH_2O(R_4)$ avec $R_3$ et $R_4$, respectivement identiques ou différents, étant des groupements acyle comprenant de 8 a 100 atomes de carbone: étant cependant entendu que l'un au moins des deux groupes Y et Z est toujours constitué par le groupe lipophile correspondant;

A et B sont soit des liaisons directes, soit des groupes, respectivement identiques ou différents, comprenant respectivement de un à trois résidus aminoacyle, eux-mêmes identiques ou différents entre eux, ou encore un groupe —NH—$(CH_2)_p$—CO— avec des valeurs de p comprises entre 2 et 10.

Dans les composés préférés de l'invention, le groupe X est un résidu aminoacide lévogyre (naturellement lorsque le résidu aminoacyle est autre que glycyle). Des groupes X particulièrement préférés sont L-alanyle, L-seryle, L-valyle, L-leucyle, L-isoleucyle, L-thréonyle ou glycyle. De préference encore $R_2$ est constitué par un groupe alcoyl-oxy.

Les groupes A et B sont avantageusement constitués par des liaisons directes, cas dans lequel le groupe Y est directement lié au gamma-carboxyle du groupe glutamyle et cas dans lequel le groupe Z est directement lié à l'atome d'oxygène en position 6 du groupe saccharidique. A et B peuvent également être constitués par un ou plusieurs résidus aminoacyle ayant les significations indiquées pour X, de préférence L'alanyle et/ou L-lysyle.

Il va de soi que dans ce qui précède, on peut avoir recours à toutes les combinaisons possibles mettant en jeu un ou plusieurs des groupes préférés qui ont été mentionnés, ces groupes pouvant être combinés, indépendamment les uns des autres, avec la structure de base du muramylpeptide modifié par le groupe portant les deux chaînes lipophiles.

Un groupe de composés est cependant particulièrement préféré. Il s'agit de ceux dans lesquels:

le groupe Z est constitué par le groupe lipophile corréspondant, tel qu'il a été défini plus haut, et dans lequel $R_2$ est un groupe alcoyl-oxy comprenant de 1 a 10 atomes de carbone, et plus particulièrement 4 atomes de carbone, notamment n-butyl-oxy,

A est une liaison directe et

Y est un groupe $NH_2$.

Ces derniers types de composés sont particulièrement remarquables en ce qu'ils sont pratiquement dépourvus de toute pyrogénicité.

Des groupes lipophiles $R_3$ et $R_4$ particulièrement préférés sont constitués par des groupes palmitoyle.

L'invention concerne plus particulièrement les compositions de liposomes ou aisément transformables en liposomes et contenant un ou plusieurs composés selon l'invention. L'incorporation des composés selon l'invention à des liposomes conduit en effet à une exaltation considérable de leur activité stimulante à l'égard des macrophages.

Il est à remarquer que la présence des deux groupes lipophiles sur deux atomes de carbone contigus du groupe glycéryle contribue à l'ancrage du muramylpeptide sur ou dans les parois lipidiques des liposomes et intervient de façon essentielle au niveau de la stabilité des liposomes eux-mêmes et de l'absence d'effet toxique ou de déstabilisation à l'égard des macrophages entrant à leur contact. Telles sont du moins les observations qui peuvent être faites dans des essais aussi bien *in vitro* qu'*in vivo.*

Bien que cela ne soit pas indispensable, on peut utiliser, pour constituer les liposomes, des lipides caractérisés par une température de transition Tm supérieure à 37°C. Cette température de transition se rapporte à la température moyenne de la transition entre l'état de "cristaux solides" et l'état de "cristaux liquides", sous laquelle lesdits lipides sont susceptibles d'exister, notamment au sein des membranes de liposomes, à des températures respectivement inférieure et supérieure à cette température Tm. L'utilisation de liposomes caractérisés par une température Tm supérieure à 37°C favorise le ciblage exclusif des macrophages. C'est à ce titre que les phospholipides formés à partir d'acides gras comprenant dans leur chaîne de 14 à 30, notamment de 18 à 24 atomes de carbone, sont préférés.

En ce qui concerne les lipides mis en jeu pour former les liposomes (ou les compositions aptes à former des liposomes), on peut se reporter à la littérature technique qui est abondante dans ce domaine. Des compositions lipidiques préférées sont celles qui mettent en jeu des phospholipides tels que la phosphatidylcholine (dérivée d'acides gras: comportant de 12 à 20 atomes, notamment de 16 à 20 atomes de carbone), la phosphatidylsérine, le phosphatidylinositol, la phosphatidyléthanolamine, le phosphatidylglycérol, et l'acide phosphatidique. Ces phospholipides peuvent être utilisés seuls ou en mélanges. Il es plus particulièrement avantageux d'avoir recours à des mélanges de distéarylphosphatidylcholine (DSPC) ou de phosphatidylcholine synthétique ou naturelle, d'une part, et de phosphatidylsérine (PS) ou phosphatidylglycérol (PG), d'autre part. Avantageusement, les liposomes sont formés, à partir de mélanges contenant les derniers phospholipides mentionnés dans un rapport de 7 volumes de DSPC ou de phosphatidylcholine (PC) à 1 à 10, de préférence 3, volumes de PS. Les activités biologiques des liposomes contenant les dérivés de l'invention se manifestent aussi bien lorsque les liposomes se présentent sous forme unilaméllaire ou plurilaméllaire. De préférence, les particules de liposomes ont des tailles supérieures ou égales a 0,1 micron, par exemple comprises entre 1 et 10 microns.

De préférence, les composés selon l'invention sont utilisés dans des liposomes ou des suspensions de ces liposomes dans des solutions aqueuses physiologiquement acceptables, de préférence stériles et isotoniques, lorsque ces compositions sont déstinées à être administrées par voie parentérale.

Il est avantageux que les suspensions de liposomes comprennent de 4 a 400 micromoles de lipides et de 20 à 200 microgrammes des dérivés lipophiles de muramylpeptides selon l'invention par millilitre de milieu.

L'invention concerne plus particulièrement encore des compositions de liposomes auxquelles sont incorporés les dérivés lipophiles de muramylpeptides à l'état lyophilisé, par conséquent parfaitement anhdyres. Il a en effet été remarqué que la lyophilisation de telles préparations de liposomes entrainait, après formation extemporanèe d'une suspension de liposomes, un fort accroissement de l'activité stimulante des macrophages. Ces compositions lyophilisées anhydres peuvent être conservées pendant plusieurs mois, en l'absence de toute perte d'activité.

La preparation des produits selon l'invention peut se faire à partir de l'acide muramique, de ses analogues ou de ses dérivés, lesquels ont en commun la structure ci-après appelée "structure muramique":

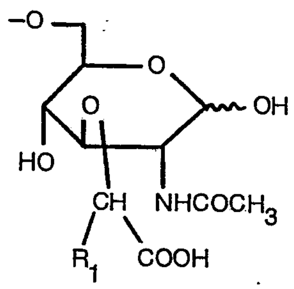

dans laquelle $R_1$ a la signification précédemment indiquée.

Des modes de préparation préférés des composés selon l'invention comprennent la fixation de la chaîne peptidique de formule X—D—Glu—(A—Y)—$R_2$, dans laquelle X, A et Y ont les significations sus-indiquées sur la structure muramique par des méthodes traditionnelles dans le domaine de la synthèse des peptides et, le cas échéant, la fixation du groupe —B—Z sur la position 6—O— de la structure muramique. Il est alors entendu que les fonctions portées par les partenaires de la reaction, et qui n'interviennent pas dans celle-ci, auront si besoin été protégées au préalable. Les fonctions protegees sont finalement libérées dans les phases terminales de la préparation des composés de l'invention. Les méthodes décrites dans la littérature antérieure et en particulier dans les demandes de brevets français dont les références sont rappelées plus loin, peuvent également être appliquées à la production des composés selon l'invention.

La substitution $R_2$ est avantageusement réalisée sur le groupe glutamyle avant la synthèse de la chaine. De même façon, le groupe Y est de préférence d'abord fixé à l'aminoacyle C-terminal avant que celui-ci ne soit intégré dans la chaine peptidique.

Les synthèses peptidiques sont réalisées suivant des méthodes traditionnelles. A titre d'exemple, on peut choisir les méthodes d'activation des carboxyles, comme la méthode des esters activés ou celle dite des anhydrides mixtes, ou bien celle utilisant un composé du type carbodiimide tel que le N,N'-dicyclohexylcarbodiimide ou des carbodiimides équivalents. On trouvera une revue des modes de synthèse peptidique traditionnels dans J. H. JONES, Chemistry and Industry, 723 (1974). On peut aussi se reporter aux demandes de brevets français déjà citées, ou encore aux demandes suivantes: n° 75 29624, 76 06819, 78 06820, 76 06821, 76 21889, 77 02646, et aux articles de LEFRANCIER et al. (Int. J. Peptide Protein Res., 1977, *9*, 249 et 1978, *11*, 289 et J. Med. Chem. 25, 1982, 87).

La formation des dérivés esterifiés ou amidés correspondant au groupe $R_2$ est obtenue de façon connue. On peut en particulier se reporter aux demandes de brevets français indiquées ci-dessus, et notamment aux demandes n° 76 06820, 76 06821, 76 21889 et 77 02646.

Lorsque le groupe Y doit être formé par un groupe glycérol porteur des deux chaines lipophiles, il est obtenu par estérification de la fonction gamma-carboxyle, le cas échéant activée, du groupe glutamique ou (lorsque A ne constitue pas une liaison directe) de l'aminoacyle C-terminal, par un glycérol lui-même béta, gamma-diestérifié par des chaines d'acides lipophiles, notamment d'acides gras, ce glycérol portant néanmoins une fonction hydroxyle terminale libre. La fonction N-terminale de la chaine peptidique aura en général été protegée, préalablement à ladite réaction d'estérification, par exemple par un groupe t-butyl-oxy-carbonyle (BOC). On peut encore se reporter au brevet français n° 78 08049, dans lequel on décrit également des modes de préparation des chaines peptidiques modifiées par un groupe glycérol, lui-même porteur d'autres groupes. Ces modes de préparation sont également applicables à la préparation des chaines peptidiques modifiées pouvant être mises en oeuvre pour la préparation des composés selon la présente invention. Ainsi peut être réalisée la synthèse de la séquence peptidique portant le groupe di-substitué lipophile.

La synthèse du dérivé de muramylpeptide correspondant est alors complétée par le couplage avec la structure muramique de la chaine peptidique (modifiée ou non, selon le cas, par le groupe glycérol di-substitué) après libération préalable de la fonction amine N-terminale. Cette structure muramique aura

elle-même été protégée au préalable. Ele sera par exemple constituée par l'acide alpha-O-benzyl-4,6-O-benzylidéne-N-acetylmuramique. Les dérivés glycopeptidiques sont finalement obtenus à l'état libre, après élimination (par hydrogénolyse par exemple) des groupements protecteurs.

Un autre mode de prépartion consiste à coupler directement le dérivé alpha substitué (par un amide ou un ester, suivant la formule générale) de l'acide N-acétyl-muramyl-L-alanyl-D-glutamique avec le groupe H—A—Y préalablement préparé, par exemple, mais de façon non limititive, en utilisant la méthode aux anhydrides mixtes.

Les dérivés selon l'invention dans lesquels le groupe B—Z— comprend un groupe glycérol-bêta, gamma-diestérifie par des acides lipophiles, notamment des acides gras, peuvent alors être obtenus par réaction des susdits dérivés glycopeptidiques — avant elimination des groupes protecteurs sur les positions 1, 4 et 6 du groupe saccharidique — avec l'acide glycérique — bêta, gamma-diestérifié correspondant.

D'autres caractéristiques de l'invention apparaitront encore au cours de la description qui suit, de l'un de ses exemples préférés, en l'absence de toute intention limitative.

## Exemple I

Synthèse de alpha(N-acétyl-muramyl-L-alanyl-D-isoglutamine), bêta, gamma-dipalmitoyl-sn-gycérol (MDP—GDP) de formule:

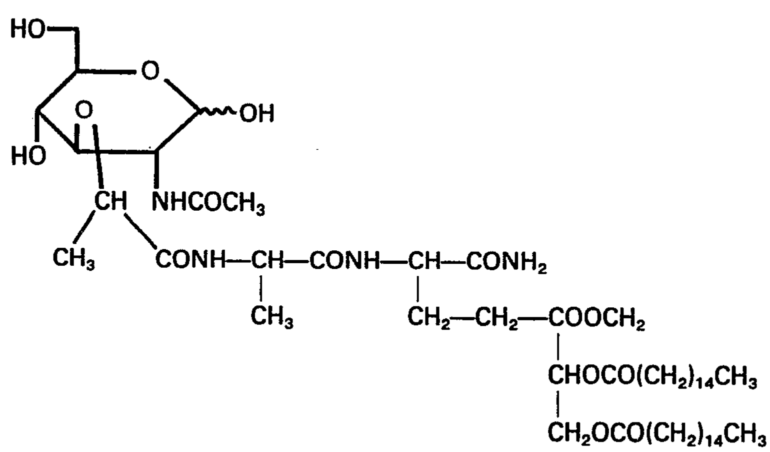

alpha-(BOC-L-Ala-D-isoGln), béta, gamma-dipalmitoyl-sn-glycérol (I):

A une solution, dans 10 ml de THF—DMF (1/1) de 119 mg (0,375 mM) de BOC-L-Ala-D-isoGln, 142 mg (0,25 mM) de bêta, gamma-dipalmitoyl-sn-gycérol et 180 mg (0,406 mM) de BOP, sont ajoutés 0,05 ml (0,406 mM) de N-méthylmorpholine et 31 mg (0,45 mM) d'imidazole. Après un week-end à température ambiante, le produit est extrait dans le chloroforme, puis purifié sur une colone de 20 g de silice 60 G, stabilisée dans du chloroforme, puis éluée avec successivement du chloroforme (100 ml), le mélange chloroforme-méthanol; 50/1 (100 ml), puis 25/1. Apres ultrafiltration de sa solution dans le chloroforme, (I) est lyophilisé à partir de sa solution dans le dioxane: 73 mg (33.6%).

alpha: 0° (c: 0,5, acide acétique glacial).

| | | | |
|---|---|---|---|
| Anal. Calc. pour $C_{48}H_{89}N_3O_{10}$%: | C: 66,4; | H: 10.3; | N: 4,8. |
| Trouvé: | C: 66,2; | H: 10,2; | N: 4,3. |

alpha-(L-Ala-D-isoGln), béta, gamma-dipalmitoyl-sn-glycérol, HCl (II):

521 mg (0,6 mM) de (I) sont traités par 1,6 ml d'une solution normale d'acide chlorhydrique dans l'acide acétique glacial, pendant 45 minutes, à température ambiante. (II) est obtenu, après concentration du solvant, par lyophilisation de sa solution dans l'acide acétique et utilisé immédiatement: 480 mg.

alpha-MurNAc-(alpha-OBzl-4,6-O-Bzi)-L-Ala-D-isoGln, béta, gamma-dipalmitoyl-sn-glycérol (III):

378 mg (0,47 mM) de (II), 260 mg (0,55 mM) de MurNAc (alpha-O-Bzl-4,6-O-Bzi), 265 mg (0,605 mM) de BOP, 0,2 ml (1,75 mM) de N-méthylmorpholine sont dissous dans 8 ml de chloroforme-DMF (5/2). Aprés 36 heures, le produit est extrait au chloroforme et purifié sur une colonne de silice stabilisée dans le mélange chloroforme-méthanol (15/1), puis éluée avec le mélange des mêmes solvants (12/1). Après ultrafiltration de sa solution dans le chloroforme, (III) est lyophilisé à partir de sa solution dans l'acide acétique: 545 mg (95%).

$[alpha]_D^{25}$: +40° (c: 0,5; acide acétique glacial).

| | | | |
|---|---|---|---|
| Anal. Calc. pour $C_{66}H_{108}N_4O_{15}$, 0,5 $CH_3COOH$%: | C: 66,2; | H: 8,8; | N: 4,5. |
| Trouvé: | C: 66,4; | H: 8,7; | N: 4,3. |

alpha(MurNAc-L-Ala-D-IsoGln), béta, gamma-dipalmitoyl-sn-glycérol (IV):

535 mg (0,44 mM) de (III) sont hydrogènés en solution dans 30 ml d'acide acétique glacial, en présence de 550 mg de Pd 5% sur charbon, pendant 40 heures. Après filtration du catalyseur, (IV) est obtenu par lyophilisation à partir de sa solution dans l'acide acétique, puis purifié sur une colonne de silice éluée par le mélange chloroforme-méthanol (5/1). Après ultrafiltration de sa solution dans le chloroforme, (IV) est lyophilisé à partir de sa solution dans l'acide acétique glacial: 348 mg (72.2%).

$[alpha]_D^{25}$: +23,4° (c: 0,7; acide acétique glacial).

| | | | |
|---|---|---|---|
| Anal. Calc. pour $C_{54}H_{98}N_4O_{15}$ 1,6 $CH_3COOH$%: | C: 60,29; | H: 9,23; | N: 4,92. |
| Trouvé%: | C: 60,3 | H: 9,2; | N: 5,0. |

Exemple II

Synthese de 6-O-(bêta, gamma-dipalmitoyl-L-glycéryl)-N-acétyl-muramyl-L-alanyl-D-glutamine-n-butyl-ester (6-O-GDP-MDPG-OnBu) de formule:

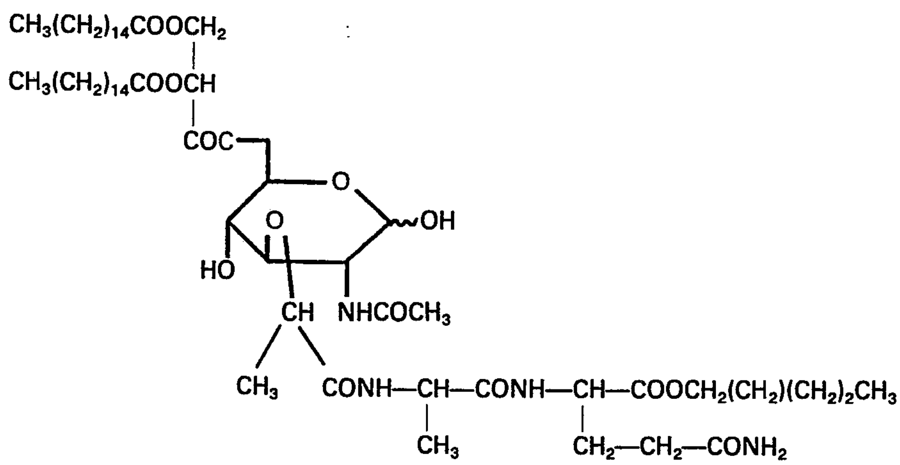

L-glycérate benzyl-ester (I):

A 222 mg (0,2 mM) d'acide glycérique, sont ajoutés 483 (2 mM) de diisopropylbenzylisourée. Après 3 heures d'agitation à température ambiante, 5 ml de THF anhydre et depéroxydè sont ajoutés; après 24 heures à température ambiante, la solution est refroidie à −15°C et la diisopropylurée est éliminée par filtration. (I) est obtenu après purification sur colonne de silice dans le mélange chloroforme-méthanol-acide acétique (120/10/1): 316 mg (80%).

$[alpha]_D^{25}$: −16,6° (c: 0,55; dioxanne).

Spectre IR: 3410—3510 $cm^{-1}$ (OH): 1735 $cm^{-1}$ (ester).

Spectre U.V.: 252 nm, 257 nm, 262 nm (benzyle).

bêta, gamma-dipalmitoyl-L-glycérate benzyl ester (II):

316 mg (1.6 mM) de (I) sont dissous dans 3 ml de pyridine sèche. A 0°C et sous courant d'argon, est ajoutée, en 15 minutes, une solution dans 6 ml de dichlorométhane sec de 1,382 g (4,83 mM) de palmitoyl chlorure. Après 4 heures de réaction à température ambiante, 80 ml d'éther sec sont ajoutés, puis 60 ml d'$H_2SO_4$ 0,5 N glacé. Apres 20 minutes d'agitation, la phase organique est lavée jusqu'à pH neutre, séchée sur $Na_2SO_4$ , filtrée et concentrée. (II) est obtenu après purification sur colonne de silice dans le mélange hexane-acétate d'éthyle (8/2): 835 mg (77,5%).

Fc: 39—40°C

$[alpha]_D^{25}$: −11,5° (c: 1; chloroforme).

| | | |
|---|---|---|
| Anal. Calc. pour $C_{42}H_{72}O_6$%: | C: 74.95: | H: 10,78. |
| Trouvé%: | C: 75,2 | H: 10,6. |

Acide bêta, gamma-dipalmitoyl-L-glycérique (III):

750 mg (1,11 mM) de (II) sont hydrogénés dans 20 ml de THF, en présence de 300 mg de Pd 5% sur charbon. Apres 3 heures, la solution est ultrafiltrée, puis concentrée jusqu'à obtention d'une poudre: 520 mg (80%).

$[alpha]_D^{25}$: −6,35° (c: 0,4; chloroforme).

| | | |
|---|---|---|
| Anal. Calc. pour $C_{35}H_{66}O_6$%: | C: 72,12; | H: 11,41. |
| Trouvé%: | C: 72,0 ; | H: 11,5. |

6-O-(bêta,. gamma-dipalmitoyl-L-gycéryl)-1-alpha-benzyl-N-acétyl-muramyl L-alanyl-D-gutamine-OnButyl ester(IV):

A une solution dans 3 ml de DMF, 658 mg (1,2 mM) de MurNAc (alpha-Bzl)-L-Ala-D-Gln-O*n*Bu, sont ajoutés 233 mg (0,4 mM) de (III), 50 mg (0,4 mM) de 4-diméthylaminopyridine, 64 mg (0,4 mM) de N-hydroxybenzotriazole, 95 mg (0, 46 mM) de DCC. Après addition de 1,5 ml de dichlorométhane sec, le mélange réactionnel est laissé sous agitation pendant 24 heures à température ambiante, puis additionné de 150 ml de chloroforme. La phase organique est lavée par HCl 0,5 M, $NaHCO_3M$, puis à l'eau, séchée sur $Na_2SO_4$ et concentrée. (IV) est d'abord précipité dans l'éthanol absolu, puis purifié sur colonne de silice éluée successivement par le mélange chloroforme-méthanol-acide acétique (18/1/0,1), par le mélange chloroforme-acide acétique (5/1), puis enfin par du méthanol. Après ultrafiltration de sa solution chloroformique, (IV) est obtenu après concentration: 330 mg (89%).

Fc: 157—162°C

$[alpha]_D^{25}$: +46° (c: 0,5; acide acétique).

Anal. Calc. pour $C_{65}H_{110}N_4O_{16}$ 0,5 $H_2O$%: C: 64,38; H: 9,23; N: 4,62.

Trouvé%: C: 64,3; H: 9,1; N: 4,4.

6-O-(bêta, gamma-dipalmitoyl-L-glycéryl)-N-acétyl-muramyl-L-alanyl-D-glutamine-OnButyl ester (V):

120 mg (0,1 mM) de (IV), dissous dans 15 ml d'acide acétique glacial et 1 ml de méthanol, sont hydrogénés en présence de 120 mg de Pd 5% sur charbon pendant 20 heures. Après filtration du catalyseur, (V) est lyophilisé à partir de sa solution dans l'acide acétique glacial: 107 mg (96%).

$[alpha]_D^{25}$: +15,4° (c: 0,4; acide acétique glacial).

Anal. Calc. pour $C_{58}H_{104}N_4O_{16}$ 1 $CH_3COOH$%: C: 61,41; H: 9,28; N: 4,77.

Trouvé%: C: 61.5; H: 9,0; N: 4,8.

## Exemple III

Synthèse de l'alpha(N-acétyl-muramyl-D-alanyl-D-isoglutamine), bêta, gamma-dipalmitoyl-sn-glycérol (MDP (D-D) — GDP).

La méthode de synthèse est la même que celle utilisée dans la préparation de MDP — *sn* GDP.

Le produit obtenu présente les caractéristiques analytiques suivantes:

$[alpha]_D^{25}$= +31,5° (c = 0,5 acide acétique glacial)

Anal. Calc. pour $C_{54}H_{98}N_4O_{13}$, 1$CH_3$ COOH (1103 546)%: C: 60,95; H: 9,31; N: 5,07.

Trouvé%: C: 60,68; H: 9,22; N: 5,17.

## Exemple IV

Synthèse de l'alpha(N-acétyl-muramyl-L-alanyl-D-isoglutaminyl-L-alanine), bêta, gamma-dipalmitoyl-sn-glycérol (MDP-L Ala-GDP).

Le procédé de synthèse est indiqué ci-dessous dans ses grandes lignes. Le produit obtenu présente les caractéristiques analytiques suivantes:

$[alpha]_D^{25}$= +15,6° (c = 0,3 acide acétique glacial)

Anal. Calc. pour $C_{57}H_{103}N_5O_{16}$, 2$CH_3$ COOH (1234 59)%: C: 59,34; H: 9,06; N: 5,67.

Trouvé%: C: 59,46; H: 9,04; N: 5,99.

## Exemple V

Synthèse de l'alpha(N-acétyl-muramyl-D-alanyl-D-isobutaminyl-L-alanine), bêta, gamma-dipalmitoyl-sn-glycérol (MDP (D-D) — L Ala-GDP).

$[alpha]_D^{25}$= +25.7° (c = 0,35 acide acétique glacial)

Anal. Calc. pour $C_{57}H_{103}N_5O_{16}$, 1,5 $CH_3$ COOH (1204 56)%: C: 59,82; H: 9,12; N: 5,81.

Trouvé%: C: 59,75; H: 9,05; N: 6,06.

Pour les deux derniers dérivés, le mode de synthèse est le suivant: préparation de BOC-L-Ala-gamma-diplamitoyl-sn-glycérol, l'estérification se faisant dans les conditions décrites précédemment pour la préparation d'alpha-BOC-L-Ala-D-isoGln-gamma-dipalmitoyl-sn-glcérol, par utilisation du couple de réactifs de couplage (BOP — imidazole).

Le groupement BOC est ensuite éliminé classiquement et le produit obtenu est couplé en utilisant le réactif de couplage BOP, avec soit le MDP, soit le MDP (D — D). Ainsi sont finalement obtenus, après une première purification sur colonne de silica, éluée avec chloroforme-méthanol 5/1, puis après une seconde purification sur colonne de LH 20, éluée avec chloroforme-méthanol 1/1, soit MDP-L-Ala-GDP, soit le MDP (D-D) L-Ala-GDP.

## Exemple VI

Synthèse du 6-O-(bêta, gamma-dipalmitoyl-L-glycérol)-ca-proyl-N-acétyl-muramyl-L-alanyl-D-glutamine-n-butyl ester (6-O-GDP-aminocaproyl-MDPG-OnBu).

Preparation des liposomes:

a) *Préparation de liposomes multilamellaires.*

Du PC ou DSPC et du PS (dans des rapports molaires respectivement de 7:3, 7:0,3 ou 7:0) sont mélangés avec du MDP-GDP, au sein d'une solution de chloroforme anhydre. La solution est introduite dans des flacons de verre à fond arrondi, soumise à une agitation rotative et simultanément à évaporation.

La pellicule de lipides anhydres formée sur la surface interne des flacons est refroidie avec une solution de chlorure de sodium tamponnée au phosphate PBS à 20°C pendant 10 minutes, la suspension étant ensuite soumise à agitation violente à 60°C dans un dispositif dénommé VORTEX, pour former des liposomes. Les proportions relatives de MDP-GDP vis-à-vis des phospholipides et les proportions relatives due tampon PBS vis-à-vis des lipides ont été chaque fois choisies de façon a obténir dans les liposomes finaux des proportions rélatives de phospholipides et de MDP-GDP, par rapport aux volumes de la suspension utilisée, qui sont indiquées dans les tableaux de résultats indiques plus loin. Des liposomes contenant du MDP ont été produits dans les mêmes conditions, en vue de réaliser des comparaisons.

Enfin des liposomes témoins ont été préparés de la méme façon, mais en l'absence des MDP-GDP.

b) *Preparation de liposomes lyophilisés.*

Des liposomes lyophilisés ont été préparés par dissolution de DSPC, de PS et de MDP-GDP, en proportions appropriées, dans du 2-méthyl-2-propanol anhydre à 28—30°C. Des parties aliquotes contenant les quantités relatives de constituants indiquées dans les tableaux de résultats qui suivent (dans un volume total de 1 ml) ont été placés dans des flacons appropriés à la lyophilisation. Après congélation des solutions de 2-méthyl-2-propanol à +4°C, les flacons ont été soumis à lyophilisation pendant 6 heures à 20°C. Les compositions lipidiques lyophilisees ont été conservées à −20°C ou +4°C dans des flacons scellés jusqu'au moment de l'emploi. La preparation de liposomes à partir de ces compositions lyophilisées peut être réalisée a tout moment par addition du volume requis de tampon PBS à 60°C, la suspension étant ensuite soumise à agitation violente dans un dispositif VORTEX pendant 2 minutes, puis conservee dans un bain de glace pendant 30 minutes avant la réalisation des essais.

Les liposomes contenant le MDP-GDP présentent une remarquable stabilité comparée à celle des liposomes fabriqués avec le MDP. Des liposomes realisés dans les mêmes conditions, mais avec des muramylpeptides marqués avec du tritium ont permis les observations suivantes. Des essais de rétention du muramylpeptide dans les liposomes mis en suspension dans un milieu MEM-FCS (mélange du "milieu essentiel minimum contenant des sels de Searle" et du sérum foetal de veau) ont montré que plus de 50% du MDP incorporé avait été perdu après 4 heures d'incubation à 37°C et que plus de 80% du MDP avait été perdu après 18 heures d'incubation. Au contraire, le MDP-GDP des liposomes correspondants avait été conservé pratiquement en totalité. L'incorporation du MDP-GDP et la stabilité de ce dernier a encore été vérifiée dans des essais de réaction avec des anticorps monoclonaux anti-MDP. Après incubation de ces liposomes dans le même milieu à 37°C pendant 72 heures, on observe une agglutination constante des liposomes avec l'anticorps (95% des liposomes étant agglutinés), cette observation témoignant de l'absence pratique de toute parte de MDP-GDP. Il a en effet été montré que la partie MDP du muramylpeptide était externalisée sur la membrane liposomale. La fuite, si elle s'était produite, aurait été apprèciée par une décroissance de l'intensité d'agglutination avec le temps. En outre, l'analyse du sucre N-acétylmuramyle par la méthode de Morgan-Elson a démontré l'absence de fuite après incubation de ces liposomes à 37°C pendant 72 heures.

Des observations tout à fait semblables ont été faites avec des liposomes préparés avec le 6-O-GDP-MDPG-OnBu ou avec les produits des exemples III, IV, V et VI.

L'invention concerne donc tout particulièrement les liposomes lyophilisés en poudre qui peuvent à tout instant être remis en suspension dans une solution tampon appropriée, par agitation dans un dispositif VORTEX pendant une durée très courte, notamment pendant 2 minutes. Dans les essais qui suivent, il sera souvent fait référence à la mise en oeuvre des "liposomes lyophilisés". Il va de soi que cette expression concerne les liposomes préalablement remis en suspension dans les conditions sus-indiquées, notamment dans un tampon PBS.

L'invention concerne plus particulièrement encore des liposomes lyophilisés de ce type qui retiennent de 20 à 2.000 microgrammes de muramylpeptides lipophiles conformes à l'invention pour 4 à 400 micromoles de lipide. De préférence, les lipides sont eux-mêmes constitués par un mélange de DSPC et de PS, dans lequel le rapport DSPC/PS varie de 7:1 à 7:10, notamment est de l'ordre de 7:3.

Essais Biologiques

1) Essais d'activite cytotoxique *in vitro* mettant en jeu une mediation de macrophages.

Les cellules cibles utilisées dans ces essais étaient constituées par des cultures de cellules de mélanome de souris B16—BL6, maintenues en monocouches, syngénéiques pour des souris C57B1/6.

Les macrophages utilisés sont des macrophages alvéolaires de rats F344 mâles de 200 g. Les macrophages sont obtenus par un lavage du poumon avec 9 × 5 ml de milieu PBS de DULBECCO, sans calcium ou magnésium, de rats anesthesiés avec 0,5 ml 5% Nembutal ip (Laboratoires Abbott S.A.) et exsanguinés par l'intermédiaire d'une canule placée dans une artère du rein. Après centrifugation, les macrophages sont remis en suspension dans du MEM (Minimum Essential Medium) contenant des sels de Earle ("Earle's Salts" commercialisés par les "Flow Laboratories S.A.") et additionné de 5% de sérum de veau foetal inactivé, de la glutamine, du pyruvate de sodium, d'acides amines non essentiels, de vitamines et d'antibiotiques (penicilline et streptomycine).

La suspension est ensuite ajustée à $5 \times 10^5$ macrophages/ml et répartie à raison de 100 microlitres par cupule dans une boîte de plastique Titertek 76—002—05. Après incubation pendant 4 heures à 37°C dans une atmosphère contenant 5% de $CO_2$, les macrophages adhèrent aux parois des cupules. Les cellules non

adhérentes sont alors éliminées par lavage poussé avec le milieu MEM-FCS. Les monocouches de macrophages sont alors incubées avec les préparations de liposomes, avec du MDP non liposomique et des liposomes témoins contenus dans un volume final de 200 microlitres pendant 24 heures. Les monocouches de macrophages sont ensuite lavées trois fois, chaque fois avec 200 microlitres du milieu MEM-FCS par cupule.

Par ailleurs, des cellules cibles B16—BL6 en phase de croissance exponentielle, ont été incubées en présence de 0,2 microcuries/ml de $^{125}$I -IdUrd (activité spécifique 2200 curies/mMole) pendant 24 heures. Les monocouches ont ensuite été lavées de façon poussée avec du MEM exempt de sérum pour éliminer tout $^{125}$I -IdUrd non incorporé. Le cellules sont ensuite détachées par traitement pendant 1 minute avec une solution de trypsine (0,25%)/EDTA (0,02%) dans du PBS. Les cellules detachées sont ensuite suspendues danns le milieu MEM-FCS. Après lavage par centrifugation à 800 g pendant 10 minutes à 4°C, les cellules ont été remises en suspension dans le milieu MEM-FCS pour obtenir une concentration de cellules viables de $5 \times 10^4$/ml.

Des aliquotes (100 microlitres de cette suspension) sont introduites dans les cupules au contact des monocouches de macrophages prétraités et une coculture est effectuée pendant des durées de 72 et/ou 96 heures respectivement. L'efficacité de revouvrement des cellules B16—BL6 s'est révélée être de l'ordre de 90—95%.

A la fin de la période d'incubation, chaque cupule a été lavée trois fois avec 200 microlitres de PBS réchauffe et les cellules adhérentes restantes ont été lysées par l'addition de 200 microlitres de NaOH 0,5 M. Le lysat et les lavages de chaque puits ont été combinés et introduits dans la cuve d'un compteur à scintillation liquide. Toutes les déterminations ont été effectuées en triple. La cytotoxicité spécifique en% à été calculée en utilisant la formule suivante:

$$\frac{\left[\begin{array}{c}\text{Comptage moyen (CPM)}\\ \text{sur cellules cibles}\\ +\\ \text{macrophages témoins}\end{array}\right] - \left[\begin{array}{c}\text{Comptage moyen (CPM)}\\ \text{sur cellules cibles}\\ +\\ \text{macrophages traités}\end{array}\right]}{\left[\begin{array}{c}\text{Comptage moyen (CPM)}\\ \text{sur cellules cibles}\\ +\\ \text{macrophaages temoins}\end{array}\right]} \times 100$$

Il est à remarquer que ce test repose sur la proportion d'ADN des cellules B16—BL6 qui est libérée dans le milieu pa lyse desdites cellules, lorsque celles-ci sont tuées par les macrophages (ou non libérée pour ce qui est des cellules B16—BL6 survivantes).

Le résultats de ces essais apparaissent dans le tableau I. On y indique les résultats de la radioactivite résiduelle des cellules traitées dans les conditions sus-indiquées (CPM ± déviation standard (SD)) cytotoxicités en% (valeurs aparaissant entre parenthèses dans le tableau I).

Dans la partie gauche du tableau apparaissent également les concentrations du MDP ou du MDP-GDP par millilitre de suspension liposomique ou de solution saline, pour ce qui est du MDP libre.

Les mêmes essais ont été réalisés avec des liposomes retenant du MDP-GDP reformés à partir de préparations de "liposomes lyophilisés". Les résultats figurent dans les tableaux II et III. Les observations suivantes découlent de l'examen de ces trois tableaux.

Le tableau I démontre que les lioposomes de MDP exercent une activité de sensibilisation à l'égard des macrophages 700 fois plus élevés que le MDP libre. Mais les liposomes de MDP-GDP (appréciés en équivalents MDP) sont 7.000 fois plus actifs que le MDP libre et par conséquent 10 fois plus actifs que les liposomes de MDP (tableau I). Mais les liposomes lypohilisés de MDP-GDP (tableaux II et III) sont considérablement plus actifs encore puisque, à doses égales, les liposomes lyophilisés de MDP-GDP ont une activité stimulante 500.000 fois plus élevée que le MDP libre (valeurs exprimées par rapport aux doses équivalentes MDP du MDP-GDP retenues dans les liposomes). L'effet de stimulation des macrophages est particulièrement important lorsque les cocultures ont été réalisées pendant 96 heures.

Cette cytotoxicité, conjuguée avec la très grande stabilité des liposomes, revêt donc une importance tout à fait remarquable, surtout si l'on se remémore que les liposomes lyophilisés, en poudre, peuvent être conservés pendant plusieurs mois sans perte d'activité.

2) Essais *in vivo.*

a) Activation in situ de macrophages alvéolaires obtenus à partir de souris auxquelles avaient été administrés des liposomes lyophilisés contenant du MDP-GDP et distribution in vivo *des liposomes contenant le MDP-GDP et le 5-O-GDP-MDPG-OnBu.*

Des liposomes lyophilisés contenant 10 microgrammes de MDP-GDP ou de 6-O-GDP-MDPG-OnBu dans un volume de 200 microlitres de PBS ont été injectés dans la veine de la queue de souris C57B1/6J. Les macrophages alvéolaires ont été récoltés 24 heures plus tard dans les conditions décrites plus haut, par lavage des poumons des animaux. Après lavage avec le tampon MEM-FCS, les macrophages ont été dosés dans les cupules des microplaques ($5 \times 10^4$ macrophages par cupule) et des cellules de mélanomes B16—BL6 marquées par la $^{125}$I-Id-Urd ont été ajoutées. Les activités cytotoxiques ont été mésurées dans les conditions décrites plus haut.

Des essais semblables ont été réalisés avec des liposomes témoins et des liposomes contenant du MDP. Les résultats figurent dans le tableau IV, tableau qui fait apparaître que les macrophages obtenus à partir des animaux qui avaient reçu les liposomes de MDP-GDP ou de 6-O-GDP-MDPG-OnBu possèdent une activité cytotoxique importante à l'égard des cellules B16—BL6, alors que les macrophages obtenus à partir des animaux ayant reçu les liposomes témoins ou les liposomes auxquels avait été incorporé du MDP se sont révélés ne posséder une activité cytotoxique que très faiblement accrue vis-à-vis de celle des macrophages obtenus a partir d'animaux non traites.

b) *Distribution des liposomes lyophilises dans les organes.*

Ces essais ont été réalisés par injection aux animaux, dans des conditions semblables à celles qui ont été indiquées plus haut, de liposomes retenant du MDP-GDP, mais dont le DSPC avait été marqué au carbone 14.

4 heures après les injections, les animaux ont été sacrifiés et les poumons, le foie et la rate ont été prelevés. Le pourcentage de radioactivité retenu au niveau de ces organes et également du sang a été évalué. Ces mesures ont été faites avec différentes proportions relatives de DSPC et de PS. Les résultats figurent dans le tableau V, lequel fait apparaître que la présence de quantités substantielles de PS montre que l'accroissement relatif des proportions de PS par rapport au DSPC favorise une localisation pulmonaire des liposomes adsorbés *in vivo.* Le rapport molaire DSPC/PS de 7:3 donne des résultats particulièrement favorables. C'est ce rapport qui a été utilisé dans le test qui suit.

c) *Effets in situ de liposomes lyophilisés retenant du MDP-GDP sur des métastases induites expérimentalement chez la souris par les cellules B16—BL6.*

Des cellules tumorales B16—BL6 en phase exponentielle de croissance ont été récoltées par trypsinisation, lavées par centrifugation et remises en suspension dans du PBS pour fournir des suspensions contenant $5 \times 10^5$ cellules viables par millilitre. 0,2 ml de cette suspension a été injecté dans une veine latérale de la queue de souris C5781/6J. Chaque souris a été traitée par voie intraveineuse avec 200 microlitres d'une solution de 0,5 micromole des susdits liposomes lyophilisés retenant 10 microgrammes de MDP-GDP aux jours 3, 5, 7, 9 et 12 après le jour de l'injection des cellules tumorales. Au jour 21, les souris ont été tuées par dislocation cervicale et le nombre de métastases pulmonaires a été enregistré sous un microscope à dissection.

Des animaux ont été traités par des liposomes témoins dépourvus de MDP—GDP.

L'effet du traitement sur le nombre de métastases a été analysé en mettant en oeuvre le test "U" de Mann-Witney.

Les résultats figurent dans le tableau VI. On y indique en particulier les nombres moyens de métastases pulmonaires repérées chez les divers groupes d'animaux traités au jour 21 suivant l'injection des cellules B16—BL6.

Les résultats font apparaître que les liposomes lyophilisés à base de MDP-GDP sont capables d'induire une réduction très substantielle des métastases, alors que des liposomes témoins ou des doses semblables de MDP libre sont sans effet sur le développement des métastases observées chez les témoins.

L'examen des tailles des métastases résiduelles après traitement fait apparaître que les métastases de diamètre inférieur à 2 mm ont été complètement éradiquées. Seules subsistaient des métastases plus importantes ayant une taille supérieure à 2,5 mm.

Ceci étant, on remarquera que les liposomes selon l'invention présentent une activité particulièrement efficace contre les métastases, surtout si l'on retient les observations qui ont été faites par KEY M.E. et Coll. (J. Natl. Canc. Inst., 69: 1189—1198, 1982) que des métastases ayant des tailles supérieures à 1 mm n'étaient pas affectées par un traitement avec des liposomes retenant du MDP.

Les résultats obtenus *in vivo* avec des composés représentatifs de la classe de composés conformes à l'invention sont également bien supérieurs à ceux qui sont obtenus, dans des conditions semblables avec des liposomes de MTP-phosphatidyl-mono-éthanolamine. Ceux-ci présentent en effet dans les mêmes tests une activité du même ordre de grandeur que les liposomes a base de MDP.

Il est également intéressant de remarquer que les produits de l'invention présentent un maximum d'activité au bout de 96 heures, d'où une durée d'action au niveau du métabolisme qui se compare favorablement également à celle des molécules de comparaison, dont l'action est beaucoup plus rapide.

Certains des composés selon l'invention présentent à doses relativement élevées une certaine pyrogenicité, dont l'impact est cependant réduit, compte tenu de l'index thérapeutique particulièrement élevé dont ils paraissent bénéficier. Ceci étant, les muramylpeptides dans lesquels le groupe de modification par les deux chaines lipophiles et fixé sur la position 6 ou groupe saccharidique et dont la chaine peptidique se termine par un groupe glutamyl-alpha-ester-gamma-amide sont particulièrement avantageux en raison de leur pyrogénicité plus réduite. En témoignent les mésures de pyrogénicité comparées éffectuées sur des groupes de trois lapins avec les deux composés plus particulierement étudiés, selon le protocole de la Pharmacopée Européenne, Vol. 2, 1971, pages 58—60. On a en effet obtenu les résultats suivants, exprimés par les élévations de température observées chez les animaux d'expérience auxquels avait auparavant été administré 1 mg/kg des composés étudiés:

| | |
|---|---|
| pour le MDP-GDP | : 1°; 0,9° et 1°C; |
| pour le 6-O-GDP-MDPG-OnBu | : 0,2°; 0,3° et 0,5°C. |

Les résultats obtenus avec des représentants de la classe de composés selon l'invention montrent que l'introduction de deux chaines lipophiles, respectivement branchées sur deux atomes contigus d'un groupe porteur couplé à un muramylpeptide rend ce dernier particulièrement apte, surtout lorsqu'il est mis sous la forme de liposomes, a activer les macrophages et à leur conférer une forte activité anatitumorale. Il et à remarquer également que les derivés lipophiles de muramylpeptides selon l'invention ont également conserve les propriétés caractéristiques du MDP, c'est-à-dire des propriétes adjuvantes et en même temps, anti-infectieuses (notamment à l'egard des klebsiella).

En particulier, l'activité adjuvante du MDP(GDP) et du B-D-GDP-MDPG-On-Bu a été testée chez la souris Swiss femelle. Au jour 0, 100 microgrammes de C474 ont été injectés avec 500 microgrammes de sérumalbumine bovine. Au jour 3D, les animaux ont reçu un rappel de 100 microgrammes d antigène seul. Les résultats suivants ont été obtenus:

| | Reponse primaire 1.21 | Reponse secondaire 1.36 | |
|---|---|---|---|
| Témoins | 1,64 | 5,09 | 2 |
| MDP | 3,97 | 8,48 | 1,7 ++ |
| MDP-GDP | 3,64 | 10,27 | 0,52++ |
| 6-O-GDP-MDPG-OnBu | 2,64 | 8,6 | 0,71++ |

++ $p<0.01$

Les titres sont exprimés en Log 2 du titre d'hémagglutination passive. Au jour 21, les sérums ont été titrés en pools; au jour 36, ils ont été titrés separément.

L'activité anti-infectieuse (élevée comme il résulte des résultats qui suivent) a été mise en évidence par administration d'une suspension de $10^4$ cellules de *Klebsiella pneumoniae,* par injection intraveineuse à des souris Swiss de 6 à 8 semaines. On indique dans les tableaux VII à IX les conditions dans lesquelles les composés testés ont été administrés et, selon les cas considérés, le nombre de souris survivantes 10 ou 15 jours plus tard, ainsi que les pourcentages de protection obsevés par rapport à des témoins.

Les observations faites peuvent être résumées comme suit:

1) Les trois produits MDP-GDP, MDP(D,D)-GDP et GDP-MDPG-OnBu ont une activité protectrice comparable vis-à-vis de l'infection par des doses léthales chez la souris de *Klebsiella pneumoniae.*

2) Des résultats semblables sont observés avec le MDP-L-Ala-GDP et le B-O-GDP-aminocaproyl-MDPG-OnBu.

3) Quand ils sont injectés dans des liposomes, le MDP-GDP est le plus actif (à 0,1 microgramme), mais les deux autres sont néanmoins plus éfficacés (à 10 microgrammes) que lorsqu ils sont injectés en suspension aqueuse.

4) Comme avec le MDP, on peut mettre en évidence un effet synergétique avec un antibiotique (gentamicine).

5) Le MDP(D,D)-L-Ala-GDP, queiqu'actif, l'est moins que le MDP-L-Ala-GDP.

6) Les expériences realisées avec Candida montrent que le MDP-GDP est actif, que cet effet est augmenté par l'incorporation dans des liposomes, et qu'il peut s'ajouter à celui de la Fungizone (Amphotéricine B).

Les résultats obtenus contre ce dernier microorganisme résultent des tableaux qui suivent.

L'invention concerne donc des réactifs biologiques qui peuvent être constitués à l'aide des composés selon l'invention. Ces réactifs sont utiles comme composés de référence ou de comparaison pour l'étude des propriétés d'activation du macrophage de composés à l'étude, plus particulièrement à l'égard de leurs propriétés tumoricides avec, notamment, une localisation pulmonaire.

L'invention est également relative à des médicaments renfermant à titre de principe actif au moins l'un des composes selon l'invention, ces médicaments étant applicables en tant qu'agents stimulants, chez le suget auquel ils sont administrés, de l'activation des macrophages. Un domaine d'application particulièrement prefère est celui du traitement d'affections tumorales et ce la prévention des proliférations et métastases tumorales. Mais le domaine d'application des médicaments selon l'invention s'étend aussi au traitement de toutes malacies inféctieuses, et en particulier contre des germes pathogènes devenus résistants aux antibiotiques. Enfin les muramylpeptides lipophiles selon l'invention peuvent également être mis en oeuvre comme adjuvants de l'immunité induite chez un hôte par un agent imunogène, notamment un principe actif de vaccin, et ce plus particulièrement lorsque l'agent immunogène est faible. On pourra se reporter aux textes des brevets antérieurs dites plus haut, pour de qui est des conditions particuliéres dans lesquelles ces adjuvants peuvent être utilisés.

Les médicaments selon l'invention peuvent être administrés à un hôte — animal ou être humain — de toute façon appropriée à l'obtention de l'effet désiré.

L'invention concerne naturellement aussi les diverses compositions pharmaceutiques, plus particulièrement a base de liposomes mettant en jeu des lipides physiologiquement acceptables, auxquelles les composés selon l'invention peuvent être incorporés, le cas échéant en association avec d'autres substances actives.

Des compositions pharmaceutiques avantageuses sont constituées par des suspensions de liposomes injectables contenant une dose efficace d'au moins un composé selon l'invention. De préférence, ces suspensions sont réalisées dans une phase aqueuse stérilisée isotonique, de préférence saline ou glucosée.

L'invention concerne plus particulièrement de telles suspensions qui sont aptes à être administrées par injections intradermiques, intramusculaires ou sous-cutanées, intraveineuses ou encore par scarifications.

L'invention concerne aussi des compositions pharmaceutiques, de préférence sous forme de liposomes, administrables par d'autres voies, notamment par voie orale ou rectale, ou encore sous des formes déstinées a venir en contact avec des muqueuses, notamment les muqueuses oculaires, nasales, pulmonaires ou vaginales.

En conséquence, elle concerne des compositions pharmaceutiques dns lesquelles l'un au moins des composés selon l'invention se trouve associé à des excipients pharmaceutiquement acceptables, solides ou liquides, adaptés à la constitution de formes d'administration orale, oculaire ou nasale ou avec des excipients adaptés à la constitution de formes d'administration rectale, ou encore avec des excipiens adaptés à l'administration vaginale, par exemple gélatineux. Elle concerne enfin des compositions destinées à la voie pulmonaire, notamment des solutions preparées en vue de l'administration au moyen d'un appareil d'aérosols classique.

A titre d'exemples de doses susceptibles d'être administrées, pour renforcer les défenses antitumorales de l'hôte, on mentionnera des doses de 10 à 10.000 microgrammes par kg de corps, par exemple de 50 à 500 microgrammes/kg lorsque l'administration est effectuée par voie parentérale, ou encore d'une dose de 1 à 10 milligrammes par kg de corps, par voie orale. Ces doses sont exprimées en équivalents MDP-GDP inclus dans les liposomes.

Ces compositons peuvent aussi être utilisées pour réaliser des injections intralésionnelles dans des tumeurs du type tumeurs mammaires, mélanomes et autres tumeurs solides.

Activité antivirale des composés selon l'invention notamment du MDP-GDP.

L'activité antivirale de cette molécule a été évaluée dans un système d'infection expérimentale de la souris par du virus grippal. Le protocole expérimental a comporté l'administration du MDP-GDP par différentes voies, notamment per os à 1 dose de 0,1 mg par souris et sous-cutanée à la même dose. L'infection des souris (souris Swiss âgées de 10 semaines) a été réalisée par inoculation intranasale de 50 microlitres d'une suspension de virus Influenza APR/8 au 1/10 000. Le MDP-GDP a été donné soit 24 heures avant l'infection (J − 1), soit 24 heures après (J + 1). L'activité antivirale a été jugée en comparant au 21ème jour après l'infection, le nombre d'animaux survivants dans les groupes traités par rapport à celui du groupe des témoinis non traités.

A la dose utilisée, une activité antivirale du MDP-GDP a été mise en évidence à titre préventif après administration tant par voie orale (56% de survie) que par voie sous-cutanée (44% de survie).

Ces résultats permettent de penser que les doses des composés selon l'invention susceptibles d'être utilisées en clinique humaine peuvent s'inscrire dans une gamme allant de 0,01 mg/kg à 0,5 mg/kg. En ce qui concerne les modalités de présentation des molécules, on doit retenir en particulier leur utilisation en suspension aqueuse ou huileuse, en émulsion huileuse ou en association avec des liposomes.

TABLEAU I

Action des muramylpeptides incorporés dans des liposomes sur l'activité cytotoxique des macrophages

| Traitement des macrophages[a] | | Radioactivité résiduelle, CPM ± SD (% cytotoxicité)[b] |
|---|---|---|
| Aucun traitement, cellules | | |
| B16—BL6 seulement | | 2218 ± 87 |
| MEM-FCS | | 2257 ± 155 |
| Liposomes témoins | | 2179 ± 79 (3,5) |
| Liposomes-MDP | 1,0 µg/ml[c] | 1290 ± 87 (43) p <0,001 |
| Liposomes-MDP | 0,1 | 1483 ± 275 (34) p <0,05 |
| Liposomes-MDP | 0,008 | 1897 ± 323 (16) |
| Liposomes-MDP-GDP | 1,0 µgl/ml | 336 ± 99 (85) p <0,001 |
| Liposomes-MDP-GDP | 0,1 | 893 ± 164 (61) p <0,001 |
| Liposomes-MDP-GDP | 0,01 | 1674 ± 115 (26) p <0,01 |
| MDP | 100 µg/ml[d] | 1501 ± 103 (34) p <0,01 |
| MDP | 33 | 1629 ± 313 (28) p <0,05 |
| MDP | 11 | 1725 ± 225 (24) p <0,05 |
| MDP | 3,8 | 1876 ± 144 (17) p <0,05 |

[a] $5 \times 10^4$ macrophages contenus dans un volume de 200 microlitres ont été prétraités avec des liposomes DSPC/PS (80 nmoles de phospholipides), des liposomes de DSPC/PS contenant du MDP ou du MDP/GDP, ou du MDP libre, pendant, 24 heures.

[b] L'activité cytotoxique a été déterminée après 96 heures de coculture des macrophages et de $5 \times 10^3$ cellules cibles B16—BL6 marquées par du $[^{125}I]$-IdUrd.

[c] Concentrations de MDP-GDP dans les liposomes.

[d] Concentrations de MDP libre.

TABLEAU II

Action des liposomes lyophilisés contenant du MDP-GDP sur l'activité cytotoxique des macrophages

| Traitement des macrophages[a] | | Radioactivité résiduelle, CPM ± SD (% cytotoxicité)[b] 72 heures | 96 heures |
|---|---|---|---|
| Aucun traitement, cellules | | | |
| B16—BL6 seulement | | 2562 ± 82 | 2331 ± 103 |
| MEM—FCS | | 2752 ± 126 | 1945 ± 217 |
| Liposomes témoins | | 2626 ± 94 (5) | 1877 ± 160 (3,5) |
| Liposomes-MDP-GDP | 18,0 µg/ml[c] | 1838 ± 168 (32) p <0,001 | 370 ± 112 (81) p <0,001 |
| Liposomes-MDP-GDP | 0,9 | 1756 ± 48 (36) p <0,001 | 312 ± 67 (84) p <0,001 |
| Liposomes-MDP-GDP | 0,09 | 1222 ± 52 (56) p <0,001 | 310 ± 83 (84) p <0,001 |
| Liposomes-MDP-GDP | 0,009 | 2094 ± 338 (24) p <0,05 | 856 ± 149 (56) p <0,01 |
| MDP | 500 µg/ml[c] | 1348 ± 113 (52) p <0,001 | 606 ± 153 (69) p <0,001 |
| MDP | 100 | 1870 ± 288 (32) p <0,01 | 1034 ± 141 (47) p <0,01 |
| MDP | 10 | 2540 ± 148 (11) p <0,05 | 1055 ± 37 (45) p <0,01 |
| MDP | 1 | 2650 ± 208 (4) | 1259 ± 284 (35) p <0,05 |
| MDP | 0.1 | 2662 ± 100 (3) | 1428 ± 88 (26) p <0,05 |

[a] $5 \times 10^4$ macrophages contenus dans un volume de 200 microlitres ont été prétraités avec des liposomes DSPC/PS (80 nmoles de phospholipides), des lisopomes de DSPC/PS contenant du MDP/GDP, ou du MDP libre, pendant 24 heures.

[b] L'activité cytotoxique a été déterminée après 72 heures ou 96 heures de coculture des macrophages et de $5 \times 10^3$ cellules cibles B16—BL6 marquées par du $[^{125}I]$-IdUrd.

[c] Concentrations de liposomes en MPD-GDP.

[d] Concentrations de MDP libre.

TABLEAU III

Influence de la concentration en MDP-GDP dans des liposomes lyophilisés le contenant sur l'induction d'une activité cytotoxique des macrophages

| Traitement des macrophages[a] | Radioactivité résiduelle, CPM ± SD (% cytotoxicité)[b] | |
|---|---|---|
| | Teneur des liposomes en phospholipides en nmoles par millilitre 400 | 40 |
| Aucun traitement, cellules | | |
| B16—BL6 seulement | 2076 ± 202 | 1962 ± 134 |
| MEM—FCS | 2214 ± 198 | 1986 ± 36 |
| Liposomes témoins | 2042 ± 56 (8) | 1900 ± 98 (4) |
| Liposomes-MDP-GDP 18,0 μg/ml[c] | 420 ± 168 (81) p <0,001 | |
| Liposomes-MDP-GDP 0.9 | 494 ± 202 (78) p <0,001 | |
| Liposomes-MDP-GDP 0,09 | 228 ± 202 (90) p <0,001 | |
| Liposomes-MDP-GDP 0.009 | 416 ± 32 (81) p <0,001 | |
| Liposomes-MDP-GDP 1,8 μg/ml | | 824 ± 162 (63) p <0,001 |
| Liposomes-MDP-GDP 0,09 | | 1170 ± 182 (41) p <0,01 |
| Liposomes-MDP-GDP 0,009 | | 1662 ± 112 (16) p <0,01 |
| Liposomes-MDP-GDP 0,0009 | | 1940 ± 46 (1) |

[a] $5 \times 10^4$ macrophages contenus dans un volume de 200 microlitres ont été prétraités avec des liposomes DSPC/PS (80 ou 8 nmoles phospholipides), ou des liposomes de DSPC/PS contenant du MDP/GDP, pendant 24 heures.

[b] L'activité cytotoxique a été déterminée après 96 heures de coculture des macrophages et de $5 \times 10^3$ cellules cibles B16—BL6 marquées par du [$^{125}$I]IdUrd.

[c] Concentrations de liposomes en MPD-GDP.

TABLEAU IVa: 1ère et 2ème séries d'expériences
Activation *in situ* de macrophages alvéolaires de la souris par des liposomes lyophilisés contenant du MDP-GDP ou du 6-O-GDP-MDPG-OnBu

| Traitement *in situ*[a] | Radioactivité résiduelle, CPM ± SD (% cytotoxicité) |
|---|---|
| *1ère expérience* | |
| Aucun traitement, cellules B16—BL6 seulement | 2032 ± 160 |
| PBS | 2019 ± 119 |
| Liposomes témoins | 1876 ± 200 (7) |
| Liposomes témoins + MDP | 1973 ± 94 (2) |
| Liposomes MDP-GDP | 1134 ± 103 (44) p <0,001 |
| *2ème expérience* | |
| Aucun traitement, cellules B16—BL6 seulement | 1638 ± 126 |
| Liposomes 6-O-GDP-MDPG-OnBu | 1364 ± 116 (33) p <0,01 |
| Liposomes témoins + MDPG-OnBu | 1606 ± 151 (2) |

[a] Des groupes de souris C57NB1/6 ont été traités avec des liposomes de DSPC/PS (0,5 µmole de phospholipide: DSPC/PS: rapport molaire de 7:3), des liposomes DSPC/PC mélangés avec 10 µg de MDP ou des liposomes DSPC/PS contenant respectivement 10 µg de MDP/GDP et de 6-O-GDP-MDPG-OnBu, chaque fois dans un volume de 200 µl, par l'intermédiaire d'une veine latérale de la queue. Les macrophages alvéolaires ont été recueillis 24 heures plus tard. Après dépôt en couche de $5 \times 10^4$ macrophages, $5 \times 10^3$ cellules B16—BL6 marquées au [$^{125}$I]-IdUrd ont été ajoutées. La cytotoxicité induite par les macrophages a été déterminée après 96 heures de coculture.
Les liposomes témoins + MDPG-OnBu ne sont pas actifs: il n'y a pas une valeur pour p.

TABLEAU IVb: 3ème série d'expériences
Activation in situ de macrophages alvéolaires de la souris par des liposomes contenant du MDP = GDP, 6-O-GDP-MDPG-OnBu ou du MDP(DD)-GDP.

| Traitement *in situ*[a] | Radioactivité résiduelle, (PM ± SD (% cytotoxicité) |
|---|---|
| Aucun traitement, cellules B16—BL6 seulement | 2360 ± 109 |
| PBS | 2301 ± 89 |
| Liposomes témoins | 2279 ± 153 (4) |
| Liposomes témoins + MDP | 2306 ± 65 (2) |
| Liposomes témoins + MDPG-OnBu | 2294 ± 128 (2) |
| Liposomes témoins + MDP(DD) | 2257 ± 149 (4) |
| Liposomes — MDP-GDP | 1534 ± 123 (35) p <0,001 |
| Liposomes — 6-O-GDP-MDPG-OnBu | 1770 ± 137 (25) p <0,01 |
| Liposomes — MDP(DD)-GDP | 1416 ± 119 (40) p <0,001 |

[a] Des groupes de souris C57B1/6 ont été traités avec des liposomes de DSPC/PS (0,5 µmole de phospholipide: DSPC/PS: rapport molaire de 7:3), des liposomes DSPC/PC mélangés avec 10 µg de MDP ou des liposomes DSPC/PS contenant respectivement 10 µg de MDP/GDP et de 6-O-GDP-MDPG-OnBu, chaque fois dans un volume de 200 µl, par l'intermédiaire d'une veine latérale de la queue. Les macrophages alvéolaires ont été recueillis 24 heures plus tard. Après dépôt en couche de 5 × 10 macrophages, 5 × $10^3$ cellules B16—BL6 marquées au [$^{125}$I]-IdUrd ont été ajoutées. La cytotoxicité induite par les macrophages a été déterminée après 96 heures de coculture.

TABLEAU V

Distribution des liposomes lyophilisés contenant du MDP-GDP[a] dans les organes

| DSPC/PS (rapport molaire)[b] | % de rétention de phospholipides[c] | | | |
|---|---|---|---|---|
| | poumon | fole | rate | sang |
| 7:3 | 8,6 ± 1,4[d] | 45,0 ± 8,4 | 2,4 ± 0,1[d] | 2,9 ± 1,3 |
| 7:0,3 | 1,2 ± 0,1 | 37,3 ± 5,0 | 2,0 ± 0,2[d] | 3,9 ± 0,8 |
| 7:0 | 1,4 ± 0,2 | 42,3 ± 1,6 | 1,6 ± 0,1 | 4,3 ± 1,6 |

[a] Des groupes de 5 souris C57B1/6 reçoivent par injection dans une veine latérale de la queue des liposomes DSPC/PS marqués par le carbone (0,5 µmole de phospholipide) contenant du MDP-GDP (10 µg) dans un volume de 200 µl. Les souris ont été sacrifiées 4 h plus tard et on a déterminé le pourcentage de rétention du marqueur $^{14}C$.

[b] Rapport molaire de DSPC au PS utilisé pour la préparation des lipsomes.

[c] La rétention du marqueur $^{14}C$ est exprimée en taut que pourcentage du marqueur par organe. Les résultats pour le sang complet sont exprimés en tant que % du marqueur radioactif par ml de sang.

[d] Différence significative vis-à-vis des liposomes de DSPC (rapport molaire 7:0), p inférieur à 0,05.

TABLEAU VIa

Action des liposomes lyophilisés contenant du MDP-GDP sur des métastases de B16—BL6[a]

| Traitement[b] | Nombre de souris | Nombre moyen de métastases pulmonaires (intervalle)[c] |
|---|---|---|
| Témoins (PBS) | 15 | 31 (19—42) |
| Liposomes témoins | 8 | 33 (18—50) |
| Liposomes-MDP-GDP, 10 µg | 8 | 8 (2—14) p <0,001[d] |
| MDP, 10 µg | 8 | 35 (22—53) |

[a] Des groupes de souris C57B16 ont reçu par injection dans une veine latérale de la queue $10^5$ cellules B16—BL6 au jour 0.

[b] Des liposomes DSPC/PS témoins (0,5 µmole de phospholipide), des liposomes de DSPC/PS contenant 10 µg de MDP-GDP ou 10 µg de MDP libre dans un volume de 200 µl de PBS ont été injectés, par l'intermédiaire d'une veine latérale de la queue, aux jours 3, 5, 7, 9 et 12 après le jour 0 de l'Injection des cellules B16—BL6.

[c] Le nombre des métastases pulmonaires a été déterminé au jour 21 après l'Injection des cellules B16—BL6.

[d] Différence significative vis-à-vis des souris témoins (test "u" de Mann-Witney).

TABLEAU VIb

Action des liposomes contenant du MDP-GDP, 6-O-GDP-MDPG-OnBu ou du PDP(DD)-GDP sur des métastases de B16—BL6.

| Traitement[a] | Nombre de souris | Nombre moyen de métastases pulmonaires (intervalle) |
|---|---|---|
| Témoins (PBS) | 10 | 67 (40—98) |
| Liposomes témoins | 9 | 73 (45—108) |
| Liposomes MDP-GDP | 9 | 31 (21—40) p <0,01 |
| Liposomes 6-O-GDP-MDPG-OnBu | 9 | 42 (18—70) o <0,05 |
| Liposomes MDP(DD)GDP | 9 | 18 (12—33) p <0,001 |

[a] Des groupes de souris C57B1/6 ont été traités avec des liposomes de DSPC/PS (0,5 µmole de phospholipide: DSPC/PS: rapport molaire de 7:3), des liposomes DSPC/PC mélangés avec 10 µg de MDP ou des liposomes DSPC/PS contenant respectivement 10 µg de MDP/GDP et de 6-O-GDP-MDPG-OnBu, chaque fois dans un volume de 200 µl, par l'intermédiaire d'une veine latérale de la queue. Les macrophages alvéolaires ont été recueillis 24 heures plus tard. Après dépôt en couche de 5 × 10 macrophages, 5 × $10^3$ cellules B16—BL6 marquées au [$^{125}$I]-IdUrd ont été ajoutées. La cytotoxicité induite par les macrophages a été déterminée après 96 heures de coculture.

TABLEAU VII

Action protectrice du MDP-GDP et des derives injectes en suspension aqueuse chez la souris infectee par klebsiella pneumoniae

| Traitement au jour -1 (i.v.) | Survie (15 j) | Protection % |
|---|---|---|
| Témoins | 1/56 | |
| MDP 100 µg | 30/56 | 52 |
| MDP-GDP 100 µg | 32/48 | 65 |
| Témoins | 0/32 | |
| MDP 100 µg | 15/32 | 47 |
| MDPG-OnBu 100 µg | 20/32 | 62.5 |
| 6-O-GDP-MDPG-OnBu 100 µg | 22/32 | 68.7 |
| Témoins | 0/24 | |
| MDP 100 µg | 12/24 | 50 |
| MDP(D,D) 100 µg | 5/24 | 20.8 |
| MDPG(D,D)-GDP 100 µg | 20/24 | 83.3 |

Souris infectée par $10^4$ K.pneumoniae

TABLEAU VIII
Action protectrice du MDP-GDP et de ses derives incorpores dans des liposomes chez la souris infectee par klebsiella pneumoniae

| Traitement au jour -1 (i.v.) | Survie (15 j) | Protection % |
|---|---|---|
| Liposomes vides | 2/24 | |
| Liposomes MDP-GDP 0.01 µg | 6/16 | 31.2 |
| 0.1 µg | 18/24 | 66.7 |
| 1 µg | 18/24 | 66.7 |
| 10 µg | 20/24 | 75 |
| Liposomes vides | 2/24 | |
| Liposomes 6-O-GDP-MDPG-OnBu 1 µg | 8/24 | 25 |
| 10 µg | 15/24 | 54 |
| Liposomes vides | 2/24 | |
| Liposomes MDPG(D,D)-GDP 1 µg | 4/24 | 8 |
| 10 µg | 14/24 | 50 |

Souris infectées par voie veineuse avec $10^4$ K.pneumoniae

TABLEAU IX
Augmentation de l'effet protecteur contre klebsiella pneumoniae par l'association de MDP ou de MDP-GDP avec une dose faible d'antibiotique

| Traitement au jour -1 | Survie (15j) | Protection (%) |
|---|---|---|
| Témoins | 0/24 | |
| Gentamicine | 5/24 | 21 |
| MDP 30 µg | 1/24 | 4 |
| MDP + gentamicine | 14/24 | 58 |
| MDP-GDP 30 µg | 4/24 | 16 |
| MDP-GDP + gentamicine | 14/24 | 58 |

Souris infectées par voie veineuse avec $5 \times 10^4$ K.pneumoniae Gentamicine 10 µg par voie sous-cutanée 2 heures après l'infection, MDP ou MDP-GDP 30 µg par voie veineuse 24 heurers avant l'infection.

N.B. Dans ces expériences l'infection est réalisée avec un nombre de bactéries plus élevé que d'habitude pour se trouver dans une situation où le MDP n'a qu'une faible action.

TABLEAU X
Action protectrice d'autres produits de la série GDP chez la souris infectée par Klebsiella pneumoniae

| Traitement au jour -1 (i.v.) | Survie (15 jours) | Protection (%) |
|---|---|---|
| Témoins | 2/16 | |
| MDP-L-Ala-GDP | 12/16 | 63 |
| Témoins | 2/16 | |
| MDP(D,D)-L-Ala-GDP 100 µ | 6/16 | 25 |
| Témoins | 0/16 | |
| 6-O-GDP-aminocaproyl-MDPG-OnBu 100 µg | 9/16 | 56 |

Souris infectées par voie veineuse par $10^4$ K.pneumoniae

TABLEAU XI
Association du MDP-GDP avec l'amphotericine B

| Traitement | Survie (10j) | Protection |
|---|---|---|
| Témoins | 0/16 | |
| Amphotéricine B 3 µg | 2/16 | 13 |
| MDP-GDP 100 µg/jour/4 jours | 3/16 | 19 |
| MDP-GDP + Amphotéricine B | 11/16 | 69 |
| MDP(D,D)-GDP 100 µg/jour/4 jours | 0/16 | |
| MDP(D,D)-GDP + Amphotéricine B | 3/16 | 19 |

Souris infectées par voie veineuse avec $2 \times 10^6$ Candida albicans; le MDP-GDP est injecté par voie veineuse à raison de 100 µg/jour pendant 4 jours (-4, -3, -2 et -1); l'amphotéricine B 3µg est injectée par voie sous-cutanée le jour de l'infection.

TABLEAU XII
Action protectrice du MDP-GDP contre une infection a candida albicans

| Traitement (jours -4, -3, -2 et -1) | Survie (10j) | Protection |
|---|---|---|
| Témoins | 0/8 | |
| MDP-GDP en suspension aqueuse (100 µg ×4) | 6/8 | 75% |
| MDP-GDP en liposomes (10 µg ×4) | 5/8 | 62.5% |
| MDP-GDP en liposomes (30 µg ×4) | 6/8 | 75% |

Souris infectées par voie veineuse avec $10^6$ Candida albicans

**Revendications**

1. Dérivés de muramylpeptides caractérisés par la formule suivante:

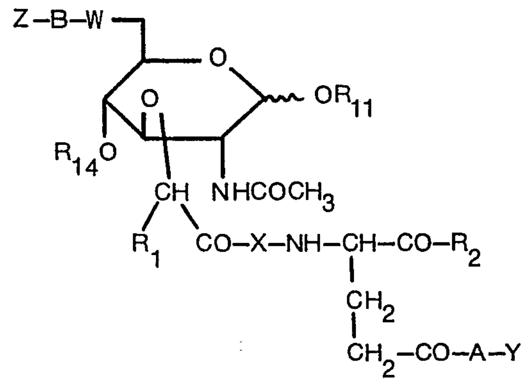

dans laquelle les substituants $R_1$, $R_2$, $R_{11}$, $R_{14}$, W, X, Y, A et B ont les significations suivantes:

$R_1$ est H ou $CH_3$,

$R_2$ est un groupe $—NH_2$, —OH ou un groupe —OV, avec V étant un groupe hydrocarboné comportant de 1 à 10 atomes de carbone,

$R_{11}$ est un atome d'hydrogène ou un groupe phénylamino,

$R_{14}$ est un atome d'hydrogène ou un groupe acyle comprenant de 1 à 4 atomes de carbone,

X et un résidu aminoacyle du groupe comprenant: alanyle, valyle, isoleucyle, norleucyle, leucyle, séryle, thréonyle, prolyle, glutaminyle, asparaginyle, méthionyle, tryptophanyle, phénylalanyle, tyrosyle, glycyle, étant entendu que ce résidue aminoacyle est éventuellement N-substitué par un groupe alcoyle inférieur, notamment méthyle;

Y a la même signification que $R_2$ ou est un groupe lipophile $—OCH_2—CHO(R_3)CH_2O(R_4)$ avec $R_3$ et $R_4$, respectivement identiques ou différents, étant des groupements acyle ou alkyle comprenant de 8 à 100 atomes de carbone;

Z a la même signification que $R_2$ ou est un groupe lipophile $—CO—CHO(R_3)CH_2O(R_4)$ avec $R_3$ et $R_4$, respectivement identiques ou différents, étant des groupements acyle ou alkyle comprenant de 8 à 100 atomes de carbone, les groupes méthylène contenus dans les groupes glycéryle, $R_3$ ou $R_4$, étant le cas échéant substitiués par un groupe alcoyle inférieur, notamment méthyle; ètant cependant entendu que l'un au moins des deux groupes Y et Z est toujours constitué par le groupe lipophile correspondant;

W est un atome d'oxygène ou, lorsque Z est le susdit groupe lipophile, un groupe NH;

A et B sont soit des liaisons directes, soit des bras de pontage, notamment des groupes, respectivement identiques ou différents, comprenant respectivement de un à trois résidus aminoacyle, eux-mêmes identiques ou différents entre eux, ou encore un groupe

$—NH—(CH_2)_p—CO—$ avec des valeurs de p comprises entre 2 et 10, ou $—NH—CH_2—CH_2—O—$, et enfin l'un des groupes méthylène du résidu glutamyle étant, le cas échéant, substitué par un alcoyle inférieur, notamment méthyle.

2. Dérivés de muramylpeptides selon la revendication 1, caractérisés par la formule générale suivante:

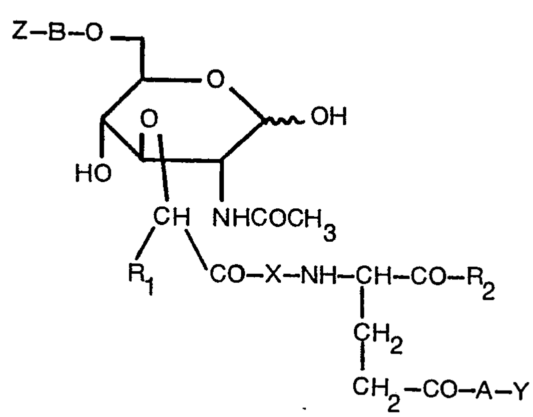

dans laquelle les substituants $R_{1,\ 2}$, X, Y, A et B ont les significations suivantes:

$R_1$ est H ou $CH_3$,

$R_2$ est un groupe $—NH_2$, —OH ou un grupe —OV, avec V étant un groupe hydrocarboné comportant de 1 à 10 atomes de carbone,

X est un résidue aminoacyle du groupe comprenant: alanlyle, valyle isoleucyle, norleucyle, leucyle, séryle, thréonyle, prolyle, glutaminyle, asparaginyle, méthinoyle, tryptophanyle, phénylalanyle, tyrosyle, glycyle,

Y a la même signification que $R_2$ ou est un groupe lipophile $OCH_2$—$CHO(R_3)CH_2O(R_4)$ avec $R_3$ et $R_4$, respectivement identiques ou différent, étant des groupements acyle comprenant de 8 à 100 atomes de carbone;

Z a la même signification que $R_2$ ou et un groupe lipophile —CO—$CHO(R_3)CH_2O(R_4)$ avec $R_3$ et $R_4$, respectivement identiques ou différents, étant des groupements acyle comprenant de 8 à 100 atomes de carbone: étant cependant entendu que l'un au moins des deux groupes Y et Z est toujours constitué par le groupe lipophile correspondant;

A et B sont soit des liaisons directes, soit des groupes, respectivement identiques ou différents, comprenant respectivement de un à trois résidus aminoacyle, eux-mêmes identiques ou différents entre eux, ou encore un groupe —NH—$(CH_2)_p$—CO— avec des valeurs de p comprises entre 2 et 10.

3. Dérivés de muramylpeptides selon la revendication 2, caractérisés en ce que les groupes $R_3$ et $R_4$ comprennent respectivement de 14 à 24 atomes de carbone.

4. Dérivés selon la revendication 3, caractérisés en ce que le groupe X est un résidu aminoacide lévogyre, sauf lorsque le résidu aminoacyle est autre que glycyle, de préference tel que L-alanyle, L-séryle, L-valyle, L-leucyle, L-isoleucyle, L-thréonyle ou glycyle.

5. Dérivés selon l'une quelconque des revendications 1 à 4, caractérisés en ce que $R_2$ est constitué par un groupe alcoyl-oxy.

6. Dérivés selon l'une quelconque des revendications 1 à 5, caractérisés en ce que que les groupes A et B sont constitués par des liaisons directes.

7. Dérivés selon l'une quelconque des revendications 1 à 5, caractérisés en ce que A et B sont constitués par un ou plusieurs résidus aminoacyle ayant une ou plusieurs des significations indiquées pour X, de préférence L-alanyle et/ou L-lysyle.

8. Dérivés selon la revendication 6, caractérisés en ce que:

le groupe Z est constitué par le susdit groupe lipophile correspondant, $R_2$ est un groupe alkyl-oxy comprenant de 1 à 10 atomes de carbone, et plus particulièrement 4 atomes de carbone, notamment n-butyl-oxy, et

Y est un groupe $NH_2$.

9. Dérivés selon l'une quelconque des revendications 1 à 8, dans lesquels $R_3$ et $R_4$ sont constitués par des groupes palmitoyle.

10. Dérivé de muramylpeptide selon la revendication 3, caractérisé en ce qu'il est constitué par l'alpha(N-acétyl-muramyl-L-alanyl-D-isoglutamine);, bêta, gamma-dipalmitoyl-sn-glycérol.

11. Dérivé de muramylpeptide selon la revendication 3, caractérisé en ce qu'il est constitué par le 6-O-(bêta, gamma-dipalmitoyl-L-glycérytl)-N-acétyl-muramyl-L-alanyl-D-glutamine-n-butyl-ester.

12. Dérivé de muramylpeptide selon la revendication 3, caractérisé en ce qu'il est constitué par l'alpha(N-acétyl-muramyl-D-alanyl-D-isoglutamine), bêta, gamma-dipalmitoyl-sn-glycérol.

13. Dérivé de muramylpeptide selon la revendication 3, caractérisé en ce qu'il est constitué par l'alpha(N-acétyl-muramyl-L-alanyl-D-isoglutaminyl-L-alanine), bêta, gamma-dipalmitoyl-sn-glycérol.

14. Dérivé de muramylpeptide selon la revendication 3, caractérisé en ce qu'il est constitué par l'alpha(N-acétyl-muramyl-D-alanyl-D-isoglutaminyl-L-alanine), bêta, gamma-dipalmitoyl-sn-glycérol.

15. Composition de liposomes ou aisément transformables en liposomes et contenant un ou plusieurs composés selon l'une quelconque des revendications 1 à 14.

16. Composition de liposomes selon la revendication 15, caractérisée en ce que les lipides constitutifs de ces liposomes sont eux-mêmes formés à partir de lipides caractérisés par une température de transition Tm supérieure à 37°C, notamment par des phospholipides formés à partir d'acides gras comprenant dans leur chaîne de 14 à 30, de préférénce de 16 à 24 atomes de carbone.

17. Composition de liposomes selon la revendication 16, caractérisée en ce que les phospholipides sont choisis, parmi les phosphatidylcholines, la phosphatidylsérine, le phosphatidylinositol, la phosphatidyléthanolamine et l'acide phosphatidique, utilisés seuls ou en mélanges.

18. Composition selon la revendication 17, caractérisée en ce que les liposomes sont formés à partir de mélanges de distéarylphosphatidylcholine (DSPC) ou diphosphatylcholine (PC) et de phosphatidylsérine (PS), dans un rapport de 7 volumes de DSPC ou de PC à 1 à 10, de préférence 3, volumes de PS.

19. Composition de liposomes selon l'une quelconque des revendications 15 à 18, caractérisée en ce que les liposomes se présentent sous forme unilamellaire ou plurilamellaire et en ce que les particules de liposomes ont des tailles supérieures ouègales à 0,1 micron, notamment comprises entre 1 et 10 microns.

20. Composition selon l'une quelconque des revendications 15 à 19, caractérisée en ce qu'elles sont formées de suspensions de ces liposomes dans des solutions aqueuses physiologiquement acceptables, de préférence stériles et isotoniques, ces suspensions de liposomes comprenant notamment de 4 à 400 micromoles de lipides et de 20 à 2.000 microgrammes des dérivés lipophiles de muramylpeptides selon l'une quelconque des revendications 1 à 9 par millilitre.

21. Compositions stables de liposomes selon l'une quelconque des revendications 15 à 18, caractérisée en ce que les liposomes sont à l'état lyophilisé.

22. Compositions de liposomes selon la revendication 21, caractérisées en ce que les liposomes

lyophilisés retiennent de 20 à 2.000 microgrammes de muramylpeptides lipophiles selon l'une quelconque des revendications 1 à 9 par 4 à 400 micromoles de lipide.

23. Compositions de liposomes selon la revendication 21 ou la revendication 22, caractérisées en ce que les lipides sont eux-mêmes constitués par un mélane de DSPC et de PS, dans lequel le rapport DSPC/PS varie de 7:1 à 7:10, notamment de l'ordre de 7:3.

24. Compositions phamaceutiques exerçant une activité stimulante à l'égard de macrophages, notamment des propriétés tumoricides des macrophages, caractérisées en ce qu'elles contiennent à titre de principes actifs un dérivé de muramylpeptide conforme à l'une quelconque des revendications 1 à 14, en association avec un véhicule pharmaceutique physiologiquement acceptable.

25. Compositions selon la revendication 23, caractérisées en ce que lesdits dérivés de muramylpeptide sont sous forme de liposomes, selon l'une quelconque des revendications 15 à 24.

**Patentansprüche**

1. Muramylpeptidderivate, gekennzeichnet durch die Formel

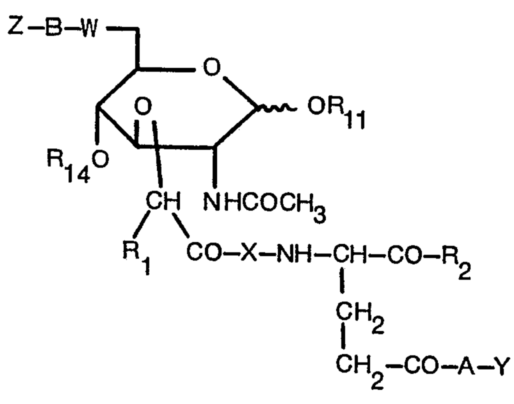

worin die Substituenten $R_1$, $R_2$, $R_{11}$, $R_{14}$, W, X, Y, A und B die folgenden Bedeutungen haben:

$R_1$ ist H oder $CH_3$,

$R_2$ ist eine $—NH_2—$, —OH— oder eine —OV— Gruppe, wobei V eine Kohlenwasserstoffgruppe mit 1 bis 10 Kohlenstoffatomen ist,

$R_{11}$ ist ein Wasserstoffatom oder eine Phenylaminogruppe,

$R_{14}$ ist ein Wasserstoffatom oder eine Acylgruppe mit 1 bis 4 Kohlenstoffatomen,

X ist ein Aminoacylrest der Gruppe bestehend aus Alanyl, Valyl, Isoleucyl, Norleucyl, Leucyl, Seryl, Threonyl, Prolyl, Glutaminyl, Asparaginyl, Methionyl, Tryptophanyl, Phenylalanyl, Tyrosyl, Glycyl, mit der Maßgabe, daß dieser Aminoacylret gegenbenenfalls durch eine niedere Alkylgruppe, insbesondere Methyl, N-substituiert ist,

Y hat dieselbe Bedeutung wie $R_2$ oder ist eine lipophile $—OCH_2—CHO(R_3)CH_2O(R_4)—$ Gruppe wobei $R_3$ und $R_4$, die jeweils gleich oder verschieden sind, Acyl- oder Alkylgruppen mit 8 bis 100 Kohlenstoffatomen sind,

Z hat dieselbe Bedeutung wie $R_2$ oder ist eine lipophile $—CO— CHO(R_3)CH_2O(R_4)—$ Gruppe, wobei $R_3$ und $R_4$, die jeweils gleich oder verschieden sind, Acyl- oder Alkylgruppen mti 8 bis 100 Kohlenstoffatomen sind, und die in den Glycerylgruppen enthaltenen Methylengruppen $R_3$ oder $R_4$ gegebenenfalls durch eine niedere Alkylgruppe, insbesondere Methyl, substituiert sind, wobei jedoch vorausgesetzt wird, daß wenigstens eine der beiden Gruppen Y und Z immer durch die entsprechende lipophile Gruppe gebildet wird,

W ist ein Sauerstoffatom, oder, wenn Z die obengenannte lipophile Gruppe ist, eine NH-Gruppe,

A und B sind entweder direkte Bindungen, oder Brückenglieder, insbesondere gleiche oder verschiedene Gruppen, die jeweils 1 bis 3 Aminoacylreste enthalten, die ihrerseits gleich oder verschieden sind, oder noch eine $—NH—(CH_2)_p—CO—$ Gruppe, wobei p Werte zwischen 2 und 10 hat, oder $—NH—CH_2—CH_2—O—$, und schließlich eine der Methylengruppen des Glutamylrests, gegebenenfalls durch ein niederes Alkyl, insbesondere Methyl, substituiert wird.

2. Muramylpeptidderivate nach Anspruch 1, gekennzeichnet durch die allgemeine Formel

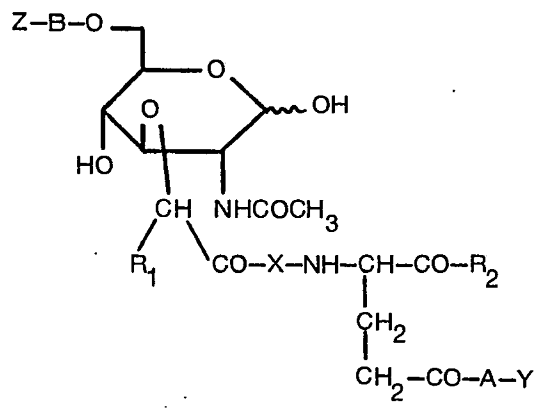

worin die Substituenten $R_1$, $R_2$, X, Y, A und B folgende Bedeutungen haben:

$R_1$ ist H oder $CH_3$,

$R_2$ ist eine $—NH_2—$, —OH— oder eine —OV-Gruppe, wobei V eine Kohlenwasserstoffgruppe mti 1 bis 10 Kohlenstoffatomen ist,

X ist ein Aminoacylrest der Gruppe bestehend aus Alanyl, Valyl, Isoleucyl, Norleucyl, Leucyl, Seryl, Threonyl, Prolyl, Glutaminyl, Asparaginyl, Methionyl, Tryptophanyl, Phenylalanyl, Tyrosyl, Glycyl,

Y hat dieselbe Bedeutung wie $R_2$, oder ist eine lipophile $—OCH_2—CHO(R_3)CH_2O(R_4)—$ Gruppe, wobei $R_3$ und $R_4$, die jeweils gleich oder verschieden sind, Acylgruppen mit 8 bis 100 Kohlenstoffatomen sind,

Z hat dieselbe Bedeutung wie $R_2$, oder ist eine lipophile $—CO—CHO(R_3)CH_2O(R_4)—$ Gruppe, wobei $R_3$ und $R_4$, die jeweils gleich oder verschieden sind, Acylgruppen mit 8 bis 100 Kohlenstoffatomen sind, wobei jedoch vorausgesetzt ist, daß wenigstens eine der beiden Gruppen Y und Z immer von der entsprechenden lipophilen Gruppe gebildet wird,

A und B sind entweder direkte Verbindungen oder Gruppen, die jeweils gleich oder verschieden sind, mit jeweils 1 bis 3 Aminoacylresten, die ihrerseits gleich oder verschieden sind, oder noch eine $—NH—(CH_2)_p—CO—$Gruppe, wobei p Werte zwischen 2 und 10 hat.

3. Muramylpeptidderivate nach Anspruch 2, dadurch gekennzeichnet, daß die Gruppen $R_3$ und $R_4$ jeweils 14 bis 24 Kohlenstoffatome enthalten.

4. Derivate nach Anspruch 3, dadurch gekennzeichnet, daß die Gruppe X ein linksdrehender Aminosäurerest ist, es sei denn, daß der Aminoacylrest nicht Glycyl ist, vorzugsweise L-Alany, L-Seryl, L-Valyl, L-Leucyl, L-Isoleucyl, L-Threonyl oder Glycyl.

5. Derivate nach einem der Ansprüche 1 bis 4, dadurch gekennzeichnet, daß $R_2$ von einer Alkyloxygruppe gebildet wird.

6. Derivate nach einem der Ansprüche 1 bis 5, dadurch gekennzeichnet, daß die Gruppen A und B durch direkte Bindungen gebildet werden.

7. Derivate nach einem der Ansprüche 1 bis 5, dadurch gekennzeichnet, daß A und B von einem oder mehreren Aminoacylresten mit einer oder mehreren der für X angegebenen Bedeutungen gebildet sind, vorzugsweise L-Alanyl und/oder L-Lysyl.

8. Derivate nach Anspruch 6, dadurch gekennzeichnet, daß die Gruppe Z von der obengenannten entsprechenden lipophilen Gruppe gebildet wird, $R_2$ eine Alkyloxygruppe mit 1 bis 10 Kohlenstoffatomen und vor allem 4 Kohlenstoffatomen, insbesondere n-Butyloxy, und daß eine $NH_2$-Gruppe ist.

9. Derivate nach einem der Ansprüche 1 bis 8, worin $R_3$ und $R_4$ von Palmitylgruppen gebildet sind.

10. Muramylpeptidderivat nach Anspruch 3, dadurch gekennzeichnet, daß es von einem α-(N-Acetyl-Muramyl-L-Alanyl-D-Isoglutamin), β,γ-Dipalmityl-sn-Glycerol gebildet wird.

11. Muramylpeptidderivat nach Anspruch 3, dadurch gekennzeichnet, daß es von dem 6-0-(β,γ,-Dipalityl-L-Glyceryl)-N-Acetyl-Muramyl-L-Alanyl-D-Glutamin-n-Butyl-Ester gebildet wird.

12. Muramylpeptidderivat nach Anspruch 3, dadurch gekennzeichnet, daß es von dem α-(N-Acetyl-Muramyl-D-Alanyl-D-Isoglutamin), β,γ-Dipalmityl-sn-Gycerol gebildet wird.

13. Muramylpeptidderivat nach Anspruch 3, dadurch gekennzeichnet, daß es von dem α-(N-Acetyl-Muramyl-L-Alanyl-D-Isoglutaminyl-L-Alanin), β,γ-Dipalmityl-sn-Glycerol gebildet wird.

14. Muramylpeptidderivat nach Anspruch 3, dadurch gekennzeichnet, daß es von dem α-(N-Acetyl-Muramyl-D-Alanyl-D-Isoglutaminyl-L-Alanin), β,γ-Dipalmityl-sn-Glycerol gebildet wird.

15. Zusammensetzung von Liposomen, oder leicht in Liposomen umzuwandelnde Zusammensetzung, enthaltend eine oder mehrere Verbindungen nach einem der Ansprüche 1 bis 14.

16. Zusammensetzung von Liposomen nach Anspruch 15, dadurch gekennzeichnet, daß die den Liposomen zugrundeliegenden Lipide ihrerseits von Lipiden gebildet und durch eine Übergangstemperatur Tm von höher als 37°C gekennzeichnet sind, insbesondere von Phospholipiden, die von Fettsäuren gebildet sind, deren Kette 14 bis 30, vorzugsweise 16 bis 24 Kohlenstoffatome aufweist.

17. Zusammensetzung von Liposomen nach Anspruch 16, dadurch gekennzeichnet, daß die Phospholipide ausgewählt sind aus den Phosphatidylcholinen, Phosphatidylserin, Phosphatidylinosit, Phosphatidylethanolamin und Phosphatidsaure, die einzeln oder gemischt verwendet werden.

18. Zusammensetzung nach Anspruch 17, dadurch gekennzeichnet, daß die Liposomen aus Gemischen von Distearylphosphatidylcholin (DSPC) bzw. Diphosphatylcholin (PC) und Phosphatidylserin (PS) erhalten werden, in einem Verhältnis von 7 Volumenteilen DSPC bzw. PC auf 1 bis 10, vorzugsweise 3, Volumenteile PS.

19. Zusammensetzng von Liposomen nach einem der Ansprüche 15 bis 18, dadurch gekennzeichnet, daß die Liposomen in einlamellärer Form oder mehrlamellärer Form vorliegen, und daß die Liposomenteilchen eine Größe von mehr als oder gleich 0,1 Mikron, insbesondere zwischen 1 und 10 Mikron, haben.

20. Zusammensetzung nach einem der Ansprüche 15 bis 19, dadurch gekennzeichnet, daß sie aus Suspensionen dieser Liposome in wässerigen Lösungen, die physiologisch verträglich sind, gebildet werden, vorzugsweise sterilen und isotonischen, wobei diese Liposomensuspensionen insbesondere 4 bis 400 Mikromol Lipide enthalten, und 20 bis 2000 Mikrogramm der lipophilen Muramylpeptidderivate pro ml gemäß einem der Ansprüche 1 bis 9.

21. Beständige Zusammensetzungen von Liposomen nach einem der Ansprüche 15 bis 18, dadurch gekennzeichnet, daß die Liposomen in einem lyophilisierten Zustand vorliegen.

22. Zusammensetzungen von Liposomen nach Anspruch 21, dadurch gekennzeichnet, daß die lyophilisierten Liposomen 20 bis 2000 Mikrogramm lipophile Muramylpeptide gemäß einem der Ansprüche 1 bis 9 auf 4 bis 400 Mikromol Lipide zurückbehalten.

23. Zusammensetzungen von Liposomen nach Anspruch 21 oder 22, dadurch gekennzeichnet, daß die Lipide ihrerseits von einem Gemisch aus DSPC und PS gebildet werden, worin das Verhältnis DSPC/PS zwischen 7:1 bis 7:10, insbesondere in der Größe von 7:3, variiert.

24. Pharmazeutische Zusammensetzungen, die eine stimmulierende Wirkung gegenüber Makrophagen ausüben, insbesondere den tumoriziden Eigenschaftern von Makrophagen, dadurch gekennzeichnet, daß sie als Wirkstoffe ein Muramylpeptidderivat enthalten, gemäß einem der Ansprüche 1 bis 14, in Verbindung mit einem pharmazeutischen Träger, der physiologisch verträglich ist.

25. Zusammensetzungen nach Anspruch 23, dadurch gekennzeichnet, daß die besagten Muramylpeptidderivate nach einem der Ansprüche 15 bis 24 als Liposome vorliegen.

**Claims**

1. Muramylpeptide derivatives, characterised by the following formula

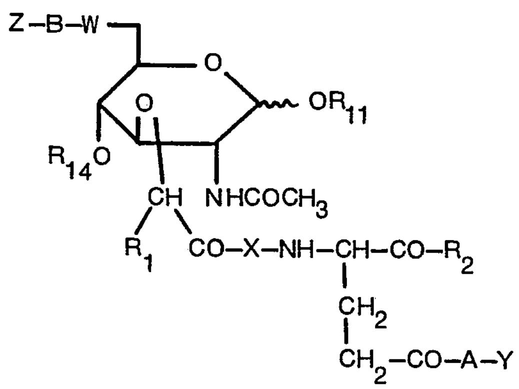

in which the substituents $R_1$, $R_2$, $R_{11}$, $R_{14}$, W, X, Y, A and B have the following meanings:

$R_1$ is H or $CH_3$

$R_2$ is a $-NH_2$, $-OH$ group, or a $-OV$ group, with V being a hydrocarbon comprising from 1 to 10 carbon atoms,

$R_{11}$ is a hydrogen atom or a phenylamino group,

$R_{14}$ is a hydrogen atom or an acyl group comprising 1 to 4 carbon atoms,

X is an aminoacyl residue of the group comprising: alanyl, valyl, isoleucyl, norleucyl, leucyl, seryl, threonyl, prolyl, glutaminyl, asparaginyl, methionyl, tryptophanyl, phenylalanyl, tyrosyl, glycyl, it being understood that said amino acyl group may be N-substituted by a lower alkyl group, more particularly methyl,

Y has the same meaning as $R_2$ or is a lipophile group $-OCH_2-CHO(R_3)CH_2O(R_4)$ with $R_3$ and $R_4$, respectively identical or different, being acyl or alkyl groups comprising from 8 to 100 carbon atoms;

Z has the same meaning as $R_2$ or is a lipophile group $-CO-CHO(R_3)CH_2O(R_4)$ with $R_3$ and $R_4$, respectively identical or different, being acyl or alkyl groups comprising from 8 to 100 carbon atoms; the methylene groups contained in the glyceryl groups, $R_3$ and $R_4$, possibly being substituted by a lower alkyl

group, more particularly methyl; it being however understood that at least one of the two groups Y and Z always consist of the corresponding lipophile group;

W is an oxygen atom or, when Z is the above mentioned lipophile group, a NH group;

A and B are either direct linkage or bridging arms, more particularly groups, respectively identical or different, comprising respectively one to three aminoacyl residue, themselves identical or different from one another, or further a $—NH—(CH_2)_p—CO—$ group with value of p comprised between 2 and 10, or $—NH—CH_2—H_2—O—$, and finally, one of the methylene groups of the glutamyl residue possibly being substituted by a lower alkyl, more particularly methyl.

2. Muramyl peptide derivatives according to claim 1, characterized by the following general formula:

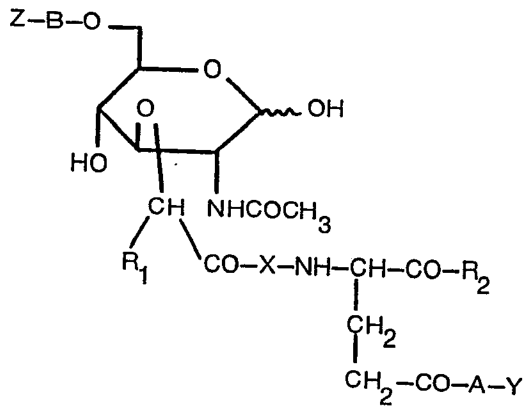

in which the substituents $R_1$, $R_2$, X, Y, A and B have the following meanings:

$R_1$ is H or $CH_3$,

$R_2$ is a $—NH_2$ or $—OH$ group, or an $—OV$ group, with V being a hydrocarbon group comprising 1 to 10 carbon atoms,

X is an aminoacyl residue of the group comprising: alanyl, valyl, isoleucyl, norleucyl, leucyl, seryl, threonyl, prolyl, glutaminyl, asparaginyl, methionyl, tryptophanyl, phenylalanyl, tyrosyl, glycyl;

Y has the same meaning as $R_2$ or is a lipophile group $—OCH_2—CHO(R_3(CH_2O(R_4)$ with $R_3$ and $R_4$, respectively identical or different, being acyl groups comprising from 8 to 100 carbon atoms;

Z has the same meaning as $R_2$ or is a lipophile group $—CO—CHO(R_3)CH_2O(R_4)$ with $R_3$ and $R_4$, respectively identical or different, being acyl groups comprising from 8 to 100 carbon atoms; it being however understood that at least one of the two groups Y and Z is always constituted by a corresponding lipophile group;

A and B are either direct linkages, or groups, respectively identical or different, comprising respectively from one to three aminoacyl residues, themselves identical or different from one another, or further a $—NH—(CH_2)_p—CO—$ group with values of p comprised between 2 and 10.

3. Muramylpeptide derivatives according to claim 2, characterised in that the groups $R_3$ and $R_4$ comprise respectively from 14 to 24 carbon atoms.

4. Derivatives according to claim 3, characterised in that the group X is a levogyratory aminoacid residue, except when the aminoacyl residue is other than glycyl, preferably such as L-alanyl, L-seryl, L-valyl, L-leucyl, L-isoleucyl, L-threonyl or glycyl.

5. Derivatives according to any one of claims 1 to 4, characterised in that $R_2$ is constituted by an alkyl-oxy group.

6. Derivatives according to any one of claims 1 to 5, characterised in that the groups A and B are constituted by direct linkages.

7. Derivatives according to any one of claims 1 to 5, characterised in that A and B are constituted by one or several aminoacyl residues having one or several aminoacyl residues having one or several of the meanings indicated for X, preferably L-alanyl, and/or L-lysyl.

8. Derivatives according to claim 6, characterised in that:

the group Z is constituted by the above-said corresponding lipophile group, $R_2$ is an alkyl-oxy group comprising from 1 to 10 carbon atoms, and more particularly, 4 carbon atoms, especially n-butyl-oxy, and Y is an $NH_2$ group.

9. Derivatives according to any one of claims 1 to 8, in which $R_3$ and $R_4$ are constituted by palmitoyl groups.

10. Muramylpeptide derivative according to claim 3, characterised in that it comprises alpha (N-acetyl-muramyl-L-alanyl-D-isoglutamine), beta, gamma-dipalmitoyl-sn-glycerol.

11. Muramylpeptide derivative according to claim 3, characterised in that it comprises 6-o-(beta, gamma-dipalmitoyl-L-glyceryl)-N-acetyl-muramyl-L-alanyl-D-glutamine-n-butyl ester.

12. Muramylpeptide derivative according to claim 3, characterised in that it comprises alpha (N-acetyl-muramyl-D-alanyl-D-isoglutamine), beta, gamma-dipalmitoyl-sn-glycerol.

13. Muramylpeptide derivative according to claim 3, characterised in that it comprises alpha (N-acetyl-muramyl-L-alanyl-D-isoglutaminyl-L-alanine) beta, gamma-dipalmitoyl-sn-glycerol.

14. Muramylpeptide derivative according to claim 3, characterised in that it comprises alpha (N-acetyl-muramyl-D-alanyl-D-isoglutaminyl-L-alanine) beta, gamma-dipalmitoyl-sn-glycerol.

15. Composition of liposomes or easily convertible into liposomes and containing one or several compounds according to any one of claims 1 to 14.

16. Composition of liposomes, according to claim 15 characterised in that the constitutive lipids of these liposomes are themselves formed from lipids characterised by a transition temperature Tm higher than 37°C, particularly by phosphilipids formed from fatty acids comprising in their chain from 14 to 30, preferably from 16 to 24 carbon atoms.

17. Composition of liposomes, according to claim 16, characterised in that the phosphilipids are selected, from among phosphatidylcholines, phosphatidylserine, phosphatidylinositol, phosphatidylethanolamine and phosphatidic acid, used alone or in mixtures.

18. Composition according to claim 17, characterised in that the liposomes are formed from mixtures of distearylphosphatidylcholine (DSPC) or diphosphatylcholine (PC) and phosphatidylserine (PS) in a ratio of 7 volumes of DSPC or of PC to 1 to 10, preferably 3, volumes of PS.

19. Compositions of liposomes according to any one of claims 15 to 18, characterised in that the liposomes are in unilamellar or plurilamellar form and the liposome particles have sizes larger than or equal to 0.1 micron, particularly comprised between 1 and 10 microns.

20. Composition according to any one of claims 15 to 19, characterised in that they are formed from suspensions of these liposomes in physiologically acceptble aqueous solutions, preferably sterile and isotonic, thee liposome suspensions comprising particularly from 4 to 400 micromoles of lipids, and from 20 to 2,000 micrograms of lipophile derivatives of muramylpeptides according to any one of claims 1 to 9 per milliliter.

21. Stable compositions of liposomes according to any one of claims 15 to 18, characterised in that the liposomes are in the lyophilised state.

22. Compositions of the liposomes according to claim 21, characterised in that the lyophilised liposomes retain from 20 to 2,000 micrograms of lipophile muramylpeptides according to any one of claims 1 to 9 per 4 to 400 micromoles of lipid.

23. Compositions of liposomes according to claim 21 or claim 22, characterised in that the lipids are themselves constituted by a mixture of DSPC and PS, in which the ratio DSPC/PS varies from 7:1 to 7:10, particularly of the order of 7:3.

24. Pharmaceutical compositions exerting a stimulant activity with respect to macrophages, particularly tumoricidal properties of macrophages, characterised in that they contain as active principals, a muramylpeptide derivative according to any one of claims 1 to 14, in association with a physiologically acceptable pharmaceutical vehicle.

25. Compositions according to claim 23, characterised in that said muramylpeptide derivatives are in the form of liposomes, according to any one of claims 15 to 24.